# EUROPEAN PATENT SPECIFICATION

(11) **EP 0 627 011 B1**
(45) Date of publication and mention of the grant of the patent: **10.09.1997**
(21) Application number: 93903760.2
(22) Date of filing: 18.02.1993
(51) Int. Cl.: C12Q 1/68, C07K 2/00

(54) **MYOTONIC DYSTROPHY**
Myotonische Distrophie
DYSTROPHIE MYOTONIQUE

(30) Priority: 18.02.1992 US 837405
(43) Date of publication of application: 07.12.1994
(73) Proprietor: UNIVERSITY OF OTTAWA, Ottawa, Ontario K1N 6N5 (CA)
(72) Inventor: KORNELUK, Robert, G., Ottawa, ON, K1G 2L8 (CA); MAHADEVAN, Mani, S., Nepean, ON, K2G 4C3 (CA)
(74) Representative: Wain, Christopher Paul
(86) International application number: CA9300068
(87) International publication number: WO9316196

(56) References cited:
- NATURE, vol. 355, 06 February 1992, London (GB); H.G. HARLEY et al., pp. 545- 546; and J. BUXTON et al., pp.547-548; and C. ASLANIDIS et al., pp. 548-551
- SCIENCE, vol. 255, 06 March 1992, Lancaster, PA (US); M. MAHADEVAN et al., pp. 1253-1255; and Y.H. FU et al., pp. 1256-1258

## Description

This invention relates to purified DNA molecules, DNA probes relating to myotonic dystrophy and procedures in which these materials are used in the detection of myotonic dystrophy in humans.

To facilitate references to some of the journal articles which provide for background in various aspects describing this invention and procedures used in carrying out the invention the following is a list by number of the journal articles:
1) G. Shutler et al , *Genomics* 13, 518 (1992)
2) G. Jansen et al., *Genomics* 13, 509 (1992)
3) C. Aslanidis et al., *Nature* 355, 548 (1992)
4) J. Buxton et al., *Nature* 355, 547 (1992)
5) H.G. Harley et al., *Nature* 355, 545 (1992)
6) P.S. Harper, *Myotonic Dystrophy* 2nd ed. (Saunders, Philadelphia 1989)
7) C.J. Höweler, H.F.M. Busch, J P.M. Geraedts, M.F. Niermeijer, A. Staal. *Brain* 112, 779 (1989)
8) I. Oberlé et al., *Science* 252, 97 (1991)
9) S. Yu et al., *Science* 252, 1179 (1991)
10) A.J.M.H. Verkerk et al., *Cell* 252, 905 (1991)
11) E.J. Kremer et al., *Science* 252, 1711 (1991)
12) L.S. Penrose, *Ann. Eugen.* 14, 125 (1948)
13) A.R. La Spada E.M. Wilson, D.B. Lubahn, A.E. Harding, K.H. Fischbeck. *Nature* 352, 77 (1991)
14) Y-H. Fu et al., *Science* 255, 1256-1258 (1992).
15) G. Jansen et al., *Nature,* submitted (1992).
16) Aslandis et al., *Nature* 355, 548-551 (1992).
17) Jansen et al., *Nature Genetics* 1, 261-266 (1992).
18) Mahadevan et al., *Science* 255, 1253-1255 (1992).
19) Harley et al., *Nature* 355, 545-546 (1992).
20) Buxton et al., *Nature* 355, 547-548 (1992).
21) Mahadevan et al., *Genomics* in press (1993).
22) Brook et al., *Cell* 68, 799-808 (1992).
23) Ghosh, *Nucleic Acids Res.* 18, 1749-1756 (1990).
24) Prestridge, *CABIOS* 7, 203-206 (1991).
25) Aslandis et al., *Nature* 355, 548-551 (1992).
26) Mahadevan et al., *Science* 255, 1253-1255 (1992).
27) Weiner et al., *Annu. Rev. Biochem.* 55, 631-661 (1986).

To facilitate reference to the myotonic dystrophy disease the short form "DM" will be used throughout the description of the invention.

Myotonic dystrophy is the most common inherited neuromuscular disease of adult life. The disease is known to affect 1 in 8,000 people worldwide. The disease is distributed worldwide with a significant prevalence in the French-Canadian population. In the Saguenay region of Quebec myotonic dystrophy affects an estimated 1 out of every 500 people based on surveys conducted by various muscular dystrophy associations. The number of diagnoses for myotonic dystrophy greatly exceeds all other muscular dystrophies including Duchenne muscular dystrophy.

There are two forms of myotonic dystrophy. The adult form usually appears in adolescence or early adulthood. The disease causes weakness and wasting of muscles, particularly of the face, jaw, neck, arms and legs. The disease also causes myotonia which is a difficulty in relaxing muscles which have been contracting for various reasons in bodily movement. In addition to these neuromuscular problems, myotonic dystrophy can also cause heart problems, digestive complications, cataracts, premature balding, mental slowness and sleep disorders.

The congenital form of myotonic dystrophy is present at birth. The infants are extremely weak, having difficulty sucking and swallowing and usually have severe respiratory problems. If the infants survive the newborn period they normally experience significant developmental delays and frequently mental retardation.

Myotonic dystrophy is an inherited disease, hence, a technique for prenatal screening would be very effective in assessing the likelihood of inheriting the disease. More specifically, myotonic dystrophy is an autosomal dominant disorder characterized as above in (6). The disease shows a marked variability in expression ranging from a severe congenital form that is frequently fatal to an asymptomatic condition associated with normal longevity. An increase in the severity of the disease in successive generations has been noted (7).

We have cloned the essential region of human chromosome 19q13 containing the myotonic dystrophy locus (1-3). We have isolated genomic and cDNA probes which map to this region and which detect an unstable 10kb *Eco*RI genomic fragment in people affected by myotonic dystrophy. The physical map location and genetic characteristics of the polymorphic segment are compatible with a direct role in the pathogenesis of myotonic dystrophy. We have now discovered that in the unstable 10kb *Eco*RI genomic fragment, the instability in this variable length polymorphism is due to variability in a trinucleotide CTG repeat. Based on this finding we are now able to provide diagnostics and procedures for determining whether or not a subject is either at risk for or is affected with myotonic dystrophy. Throughout the specification reference to the trinucleotide repeat will continue to be CTG, even though it is understood that this trinucleotide includes all analogues thereof, such analogues including in 5'-3' direction, TGC, GCT and other opposite strand entities of CAG, AGC and GCA.

Nature, vol. 355, pages 545-546 also refers to a trinucleotide repeat CAG in human genetic diseases. The trinucleotide CAG repeat is found in Kennedy's disease

In accordance with an aspect of the invention, a purified nucleic acid molecule comprises a nucleotide sequence of the type identified in Figure 1b or in Table I.

According to another aspect of the invention, a purified nucleic acid molecule comprises a variable length polymorphism within a genomic fragment of a mammalian gene isolated from human chromosome 19q13 containing a locus associated with myotonic dystrophy, the nucleic acid molecule having the restriction map of Figure la or the nucleotide sequence of Table I.

According to another aspect of the invention, a purified gene mapping to human chromosome 19q13 associated with or causative of myotonic dystrophy comprises a variable CTG trinucleotide repeat in a portion of the gene.

According to another aspect of the invention, a DNA probe is provided which comprises an oligonucleotide containing a trinucleotide repeat of at least 4 times, the trinucleotide being CTG.

According to another aspect of the invention, a DNA probe comprises an oligonucleotide comprising a sequence of at least 12 nucleotides selected from the nucleotide sequence of Figure 1b or the nucleotide sequence of Table I.

According to another aspect of the invention, a DNA probe comprises an oligonucleotide comprising a sequence of at least 12 nucleotides selected from the DNA sequence having the restriction map of Figure la or the nucleotide sequence of Table I.

According to another aspect of the invention, a method for screening a subject to determine if the subject is at risk for or affected with myotonic dystrophy, comprises:
i) providing a biological sample containing DNA or RNA of said subject to be screened,
ii) conducting a biological assay on the sample to detect the degree of expansion of a CTG trinucleotide repeat in an exon of the gene associated with myotonic dystrophy and derived from human chromosome 19q13 of said biological sample, and
iii) assessing the frequency of the trinucleotide repeat to determine at least susceptibility to myotonic dystrophy.

According to another aspect of the invention, a kit for assaying for the presence of an expanded variable length polymorphism having a CTG trinucleotide repeat in a gene associated with myotonic dystrophy and derived from human chromosome 19q13 of genomic DNA, comprises:
i) a nucleotide probe which is capable of hybridizing to target genomic DNA or its corresponding RNA copy containing the variable length polymorphism,
ii) reagent means for detecting hybridization of the probe to the polymorphism,
the reagent means and the probe being provided in amounts sufficient to effect the assay.

Preferred embodiments of the invention shall be discussed in connection with the drawings in which:

Figure 1a is a restriction of map of the genomic segment containing the variable length polymorphism in a portion of the myotonic dystrophy gene.

Figure 1b is the nucleotide sequence of the 3' region of a cDNA clone containing the variable length polymorphism.

Figure 2 is a Southern Blot analysis showing the *Eco*RI allele sizes of 9kb and 10kb and the varying degrees of allelic expansion of the 10kb allele in myotonic dystrophy chromosomes.

Figures 3 is a polyacrylamide gel electrophoresis demonstrating that the sizes of normal alleles correspond to variability in the number of CTG repeats in chromosomes from normal individuals.

Figure 4 is a Southern Blot analysis of PCR amplified genomic DNA for normal and myotonic dystrophy affected individuals.

Figure 5 is the structural organization of the human DM kinase gene with respect to the identified endonuclease restriction sites.

Figure 6A and B are Southern blot analysis of PCR amplified sequence polymorphism in the DM kinase gene.

Figure 7 is a portion of a DNA sequence containing deletion polymorphisms.

In accordance with the information disclosed in references 3 through 5, genomic and cDNA probes mapping to a 10kb *Eco*RI genomic fragment were used to detect the subject variable length polymorphism in DM individuals. We have found that the increased DM allele sizes for the variable length polymorphism (VLP) is due to an increased number of trinucleotide CTG repeats found in the 3' region of the last exon of the DM gene located within the polymorphic segment. Although the mutation has been located in this area of the gene, it is understood that the mutation could also be located in an exon of another gene in the chromosome where the other gene overlaps the subject gene. We have found that the DM gene (normal) encodes for a protein with putative serine-threoine protein kinase activity. We have also found that an increase in the severity of the disease in successive generations is related to an increase in the number of trinucleotide repeats in the DM gene. We have determined that CTG repeats in the DM gene of up to 40 constitute a normal DM gene. Repeats in excess of 40 and certainly in excess of 50 constitute a mutant gene. In some of our investigations we have located repeats of 2000 or more. The discovery of the CTG repeat allows us to conduct diagnosis to determine whether or not an individual is at risk for or is affected with muscular dystrophy.

It is understood that the term at risk for means that the infant, child or adult is predisposed to getting DM even though at the time of diagnosis there are no visible symptoms of DM. The CTG repeat may be detected by a variety of assays and in particular, the expansions are detected by Southern Blot analysis of restricted genomic DNA, by various amplification procedures such as PCR based oligonucleotide hybridization assay and the ligase chain reaction which may be used in combination with Southern Blot analysts of the amplified material.

The combined use of amplification procedure in combination with a Southern blot analysis is a particularly beneficial advantage of this invention when large CTG repeat polymorphisms are being detected. With larger repeats, an amplification procedure may not be very reliable in detecting the extent of the repeat. The subsequent Southern blot analysis of the amplified portion does however provide the additional information necessary to determine the extent of repeats in the larger polymorphisms.

The PCR technique is described in Saiki et al., *Science,* 230:1350-1353 (1985) and Saiki et al., *Nature,* 324:163-166 (1986). The ligase chain reaction technique is described in Weiss, R., *Science* 254:1292-1293 (1991). Based on the results of our investigations it is clearly apparent that DM is caused by mutations in the DM gene which generate an amplification of the specific CTG repeat in the variable length polymorphism of the gene. In order to facilitate discussion of various embodiments of the invention, it is understood that the terms nucleotide sequence, DNA sequence and RNA sequence are used interchangeably, where appropriate. It is also understood that a nucleotide sequence is intended to include a double stranded or single stranded DNA or corresponding RNA sequences. Furthermore, the sequences are purified to a level such that there is essentially complete homogeneity in the isolated gene sequence and does not include extraneous cell wall matter and other extraneous human body fluids and materials.

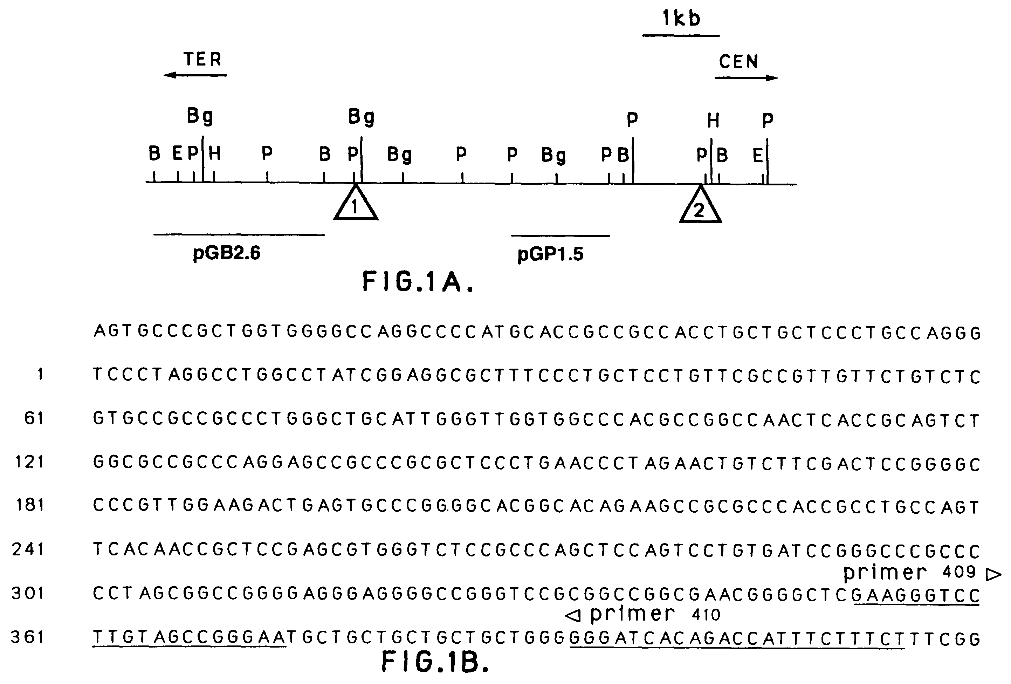

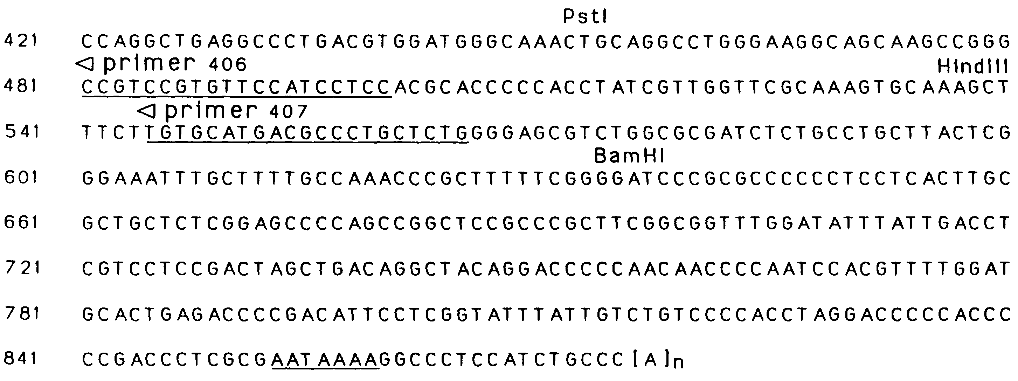

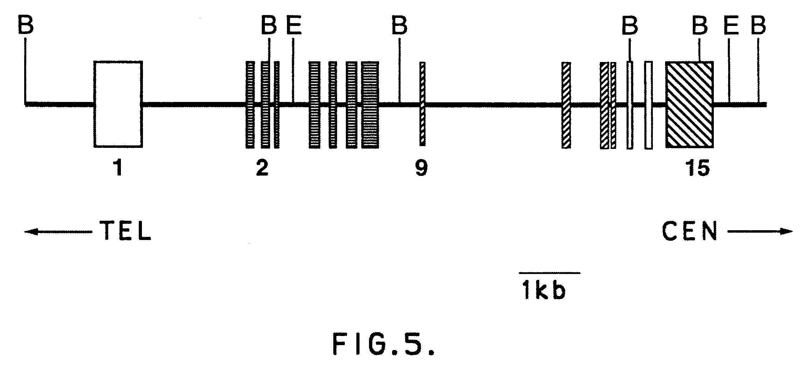

The map of Figure 1a, was constructed by the analysis of subclones derived from the cosmid Y100263, a genomic clone previously described (3). The normal insertion polymorphism and the variable length polymorphism (triangles 1 and 2 respectively) were detected by two unique sequence probes, pGB2.6 and pGP1.5. The orientation of the map is given from telomere (TER) to centromere (CEN) and the position of relevant restriction sites are given: B, *Bam*HI; *Bg*, *Bg*II; E, *Eco*RI; H, *Hind*III; P, *Pst*I*.* The nucleotide sequence of a portion of a cDNA clone is shown in Fig. 1b. The clone was isolated from a human heart cDNA library (Stratagene, Inc.) using as a probe, a 1.5kb *Bam*HI fragment containing the variable length polymorphism which is later described. The sequence shown in Fig. 1b is the last 60bp of Exon 14 (top line) followed by the entire 875bp sequence of Exon 15 at the 3' end of the gene. The nucleotide position designated by the number 1 corresponds to the second base of the Exon 15 as shown in Figure 5. Exon 15 begins at bp position 12854 of Table I.

A consensus, polyadenylation signal sequence is underlined at the lower portion of the sequence. Relevant restriction enzymes are shown in italics above their recognition sequences. Primers used for sequencing and PCR amplification are underlined and numerically designated. Indicated in bold letters are the 5'-CTG repeats found in this particular cDNA. Genomic sequence derived from cosmid clones F18894 and Y100263 is identical to the sequence of the last exon of the cDNA except there are 20 and 11 CTG repeats, respectively. This indicates an homology in the sequence except for the number of repeats in the CTG region. Plasmid DNA was prepared by a routine alkaline lysis method in order to develop the sequence. Dideoxy chain termination reactions using vector primers and internal primers were performed with fluorescent dye-labelled dideoxynucleotides, according to manufacturer's specifications (Applied Biosystems) and subsequently run on an ABI 373A automated sequencer. DNA sequencing was also performed with radioactive nucleotides to resolve sequence ambiguities.

By the use of Southern Blot analysis, two different DNA polymorphisms mapping within the 10 kb *Eco*RI genomic fragment (Fig. 1) have been identified by 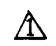 and 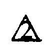 . The polymorphisms are detected by two sequence genomic probes (pGB2.6 and pGP1.5) unique to the human genome and mapping to this *Eco*RI fragment (3). The two unique sequence genomic probes pGB2.6 and pGP1.5 can be isolated from a human genomic library by using an probe homologous to the sequence presented in Fig. 1b or a portion thereof. Such a probe is used to isolate clones containing a 9kb *Eco*RI genomic fragment, with the sequence having a restriction map of the region depicted in Fig. 1a or a polymorphic variant of the 10kb *Eco*RI fragment with a sequence and restriction map identical to Fig. 1a, except for an insertion of 1 kb at the position designated by 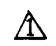 . This *Eco*RI fragment(s) can be subcloned in a standard vector.

After digestion of the *Eco*RI subclone with the restriction enzyme *Pst*I, the probe pGP1.5, a 1.5kb *Pst*I fragment can be separated by gel electrophoresis and isolated from the gel for probe use or for subcloning. Similarly, the 2.2 kb *Bam*HI/*Eco*RI fragment of the 2.6 kb *Bam*HI probe pGB2.6, can be isolated by digestion of the *Eco*RI subclone with a combination of *Bam*HI and *Eco*RI*.* These probes can be used to detect the insertion polymorphism and the variable length polymorphism associated with myotonic dystrophy.

In normal individuals, these probes detect a 8.5/9.5 kb *Hind*III or a 9.0/10.0 kb *Eco*RI insertion polymorphism. This variation is due to a 1 kb insertion located within a 150 bp *Pst*I/*Bg*II fragment (Figures 1a and 1b). The second DNA polymorphism maps to a 1.5 kb *Bam*HI fragment located 4.0 kb centromeric to the insertion polymorphism, and is associated exclusively with DM chromosomes. By way of a DNA sequence analysis of this genomic fragment we discovered a trinucleotide CTG repeat located approximately 300 bp distal to the centromeric *Bam*HI site (Fig. 1).

The 1.5 kb *Bam*HI fragment of Figure 1 as well as other genomic probes from the DM region were used to isolate human cDNAs from brain, muscle and heart libraries. The *Bam*HI fragment includes at least sequence nucleotides of Figure 1b from nucleotide portions 1 through 639. Sequence analysis shows that some of the isolated cDNAs contain the variable CTG repeat (Fig. 1). Multiple stop codons are found in all three reading frames from the 5' end to the trinucleotide repeat. Furthermore, a polyadenylation signal and a poly-A tail are present in these clones approximately 500bp downstream of the repeat region. This indicates that the CTG repeat is located within the 3' untranslated region of this particular DM gene.

The relationship of both DNA polymorphisms 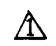 and 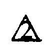 of Figure 1 to DM by Southern Blot analysis has been investigated. Genomic DNA from a total of 140 normal and 258 affected individuals from 98 DM families were used in our investigations. The insertion polymorphism found in normal individuals can be detected using a variety of restriction enzymes (as deduced from Fig. 1). In all of our affected families, the DM chromosome was found to segregate with the larger insertion allele, which has a frequency of p-0.60 in the normal population. This strong linkage disequilibrium suggests a limited number of DM mutations.

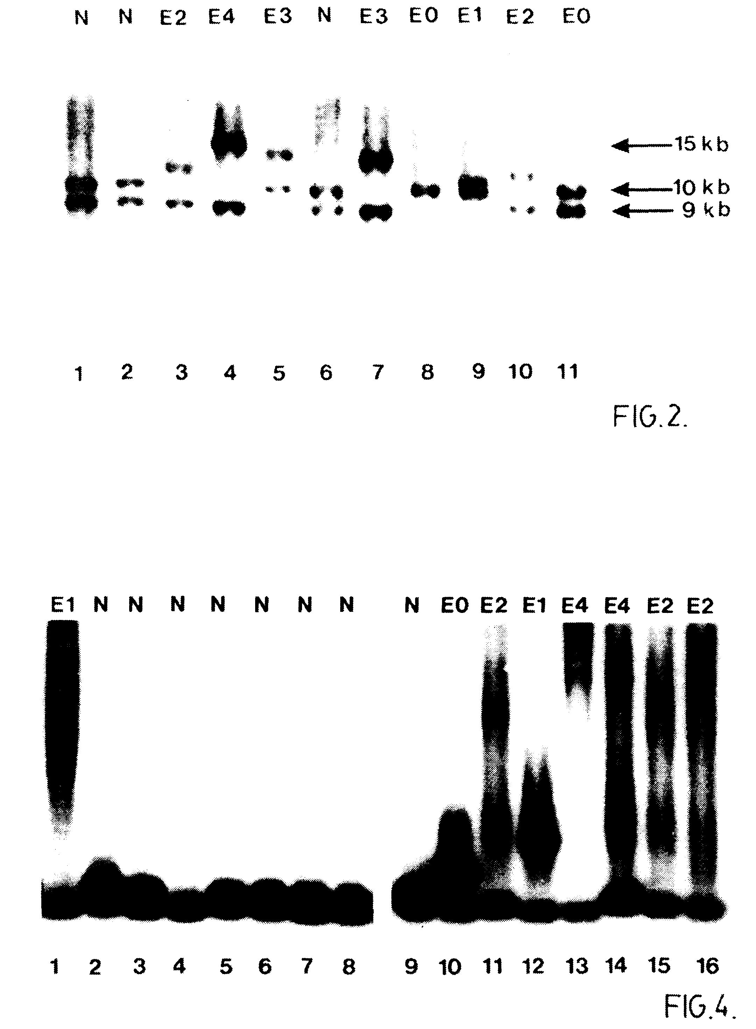

As already noted, the expansion of the variable length polymorphism in the genomic DNA of DM individuals has been previously reported (3-5). We have established that increases in the size of the enlarged fragments, up to 10 kb, are possible. For example, according to this embodiment an increase of approximately 6kb is shown in Figure 2. Nearly 70% of DM individuals (180/258) show distinct increases in the size of this fragment using Southern Blot analysis. Furthermore, almost all DM families (96/98) have at least one individual with expanded DM alleles. Normal individuals (280 analyzed) do not exhibit allelic expansion.

A Southern Blot analysis was conducted to demonstrate varying degrees of allelic expansion in the DM - affected individuals. *Eco*RI allele sizes of 9 kb and 10 kb are found in the normal population (lanes 1, 2, and 6). Genetic phasing of all the DM families reveals that the 10 kb allele is the expanding allele in DM individuals. Classification of expansion as shown in Figure 2 is as follows: E0, no expansion; E1, expansion of 0 - 1.5 kb; E2, expansion of 1.5 - 3.0 kb; E3, expansion of 3.0 - 4.5 kb; E4, expansion of 4.5 - 6.0 kb, or more. Approximately 30% of DM individuals (affected status as verified by DNA typing) show no allelic expansion on the Southern Blot level (lanes 8 and 11). Varying degrees of expansion are seen in other DM individuals ranging from E1 (lane 9) to E2 (lanes 3 and 10) to E3 (lanes 5 and 7) to E4 (lane 4). N refers to normal individuals. A variety of biological samples containing DNA, including (but not exlusively) whole blood, hair, skin, amniocyte, chrionic villi, buccal mucosa and tissue samples can be utilized in biological assays. Typically a whole blood sample is obtained from the patient by venipuncture. The white blood cells are separated from the red blood cells by centrifugation. DNA is subsequently extracted and purified by a wide range of standard techniques from the white blood cells. (Sambrook, J. E.F. Fitsch and T. Maniatis. 1989. Molecular cloning: A Laboratory Manual. Cold Spring Harbour, Laboratory Press, Cold Spring Harbour, N.Y.). The method employed in carrying out the standard Southern Blot includes digesting the extracted genomic DNA (5 µg) with *Eco*RI*.* The digested DNA is electrophoresed on 0.8% agarose gels, and transferred to nylon membranes. Southerns were probed with the *Bam*HI/*Eco*RI fragment of pGB2.6, washed at 0.2XSSC - 0.1% SDS at 65°C, and exposed to X-ray film for 1-4 days. The pGB2.6 probe was cleaved by a combination of *Bam*HI and *Eco*RI to give a 2.2 kb fragment which as shown in Figure 1a will only detect the variable *Eco*RI fragment between the two *Eco*RI sites.

Expanded alleles often have a blurred appearance on Southern Blots as shown in Fig. 2. This suggests a somatic cell heterogeneity in the size of the enlarged alleles similar to that seen in the fragile X mutation (8-11). Furthermore, preliminary results suggest there is a tendency for the DM alleles to increase in size in successive generations of a family and that the greater the expansion of these alleles, the greater the clinical severity of the disease. Therefore, genetic anticipation may, in fact, be a real phenomenon in DM (7), and may not be due to ascertainment bias, as originally suggested by Penrose (12).

To determine whether an increased number of CTG repeats is responsible for allelic expansion, PCR primers flanking the repeat region were derived from the DNA sequence depicted In Fig. 1.

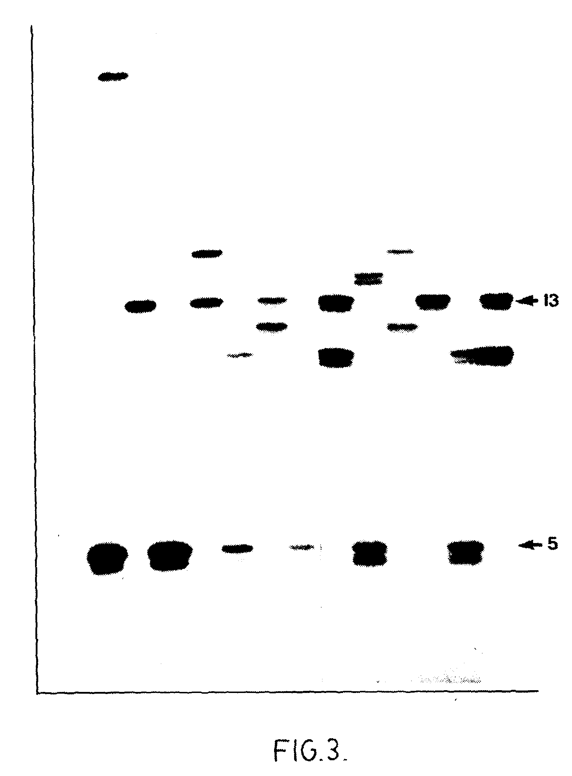

The primers may be selected from the sequences of the restriction fragment of Figure 1a or from portions of the specific listing of Figure 1b. In particular suggested primers are identified as forward primer 409 and reverse primers 406, 407 and 410. It is appreciated that the sequence of each of the reverse primers is the reverse complement of the sequence listed. All primer combinations produce similar patterns of amplification in normal individuals. A substantial variability in the size of normal alleles can be seen with agarose gel electrophoresis and ethidium bromide staining. Polyacrylamide gel electrophoresis (Figure 3) reveals that the sizes of normal alleles correspond to variability in the number of CTG repeats, ranging from 5 to 30 (124 normal chromosomes analyzed). CTG repeat numbers of 5 and 13 are the most common in the normal population frequencies of 35% and 19% respectively. The overall heterozygosity amongst normal individuals is 81%.

An analysis of ³²P-labelled PCR products by denaturing acrylamide electrophoresis shows repeat lengths ranging from 5 to 28 repeats (Fig. 3). The most common repeats in the normal population (5 repeats, 35%; 13 repeats, 19%) are designated by the arrows. Primer 409 (1 ug) was radioactively labelled using γ[³²P-ATP]. CTG repeats were amplified using genomic DNA from various normal individuals in standard PCR reactions containing 50 ng of both forward primers 409 and reverse primers 410, and 10 ng of radiolabelled primer 409. In order to provide the above results of Figure 3, the amplified products were resolved by gel electrophoresis on 8% polyacrylamide. Dried gels were exposed to Kodak X-AR5 film and autoradiographed at room temperature for 17 hours. Sequencing reactions generated from a control template were used as molecular weight standards.

PCR amplification of the CTG repeat region of genomic DNA from DM affected individuals reveals that although the unaffected alleles are readily amplified, the mutant alleles are not usually visible using ethidium bromide staining and agarose gel electrophoresis. In order to determine whether the mutant alleles were in fact amplified, a synthetic (CTG)₁₀ oligonucleotide was used to probe a Southern Blot of PCR products.

Analysis of PCR amplified genomic DNA from normal (N) and DM affected (E) individuals was conducted. Southerns were probed with a (CTG)₁₀ oligonucleotide. PCR amplified DNA from DM individuals shows a distinct smearing of the hybridization signal, presumably due to heterogeneity of the expanded allele derived from genomic DNA gathered from the biological sample. In contrast, the oligonucleotide probe hybridized only to the normal alleles in unaffected individuals. Individuals who show no expansion (E0) at the Southern Blot level, show clear expansion using the PCR based assay (lane 10). In some PCR amplified samples from DM individuals, the hybridization smear produced by the labelled (CTG)₁₀ oligonucleotide is quite faint and could be mistaken for that of an unaffected individual (lane 13). However, examination of Southern Blot analysis of *Eco*RI or *Hind*III digested genomic DNA probed with pGB2.6 or pGP1.5 reveals the presence of a greatly expanded allele (E4) in this individual. Presumably, a greatly increased number of CTG repeats is refractory to PCR amplification under the conditions used. Thus, appropriate molecular diagnosis of DM preferably includes both Southern Blot analysis of genomic DNA and the hybridization analysis of PCR amplified DNA.

The procedure used to develop the results in Figure 4 is as follows. Two µg of extracted genomic DNA as previously discussed was PCR amplified using primers 406 and 409 using a standard protocol and 30 cycles of 94°C for 1 min., 60°C for 1 min, 72°C for 1.5 min. Amplified products were electrophoresed an 1% agarose gels, transferred onto nylon membranes and probed with a labelled (CTG)₁₀ oligonucleotide. Membranes were washed at 6x SSC, 0.1% SDS, 40°C and exposed to X-ray film overnight.

Comparing human and murine DM kinase gene sequences with each other and with known cDNA sequences from both species, led to the discovery of a translation initiation codon, as well as the organization of the DM kinase gene (Mahadevan, Mani S., "Structure and Genomic Sequence of the Myotonic Dystrophy (DM Kinase) Gene", to be published in Human Molecular Genetics, Vol. 2, No. 3 (March 1993)).

To determine the structure and genomic sequence of the human DM kinase gene, a detailed restriction map of the relevant regions of the chromosome 19 cosmid clones Ya100263 and F18894 (16), was obtained. Similarly, the relevant regions of the mouse genome were mapped (17). The DM kinase gene mapped within a region of approximately 14 kb contained within five continguous BamHI fragments in cosmid Ya100263. The sequence of 13747 bp (Genbank Accession No. L08835) begins from the most telomeric BamHI site of the five BamHI fragments and ends at the position of the poly A tail of the cDNA as shown in Figure 5. It contains the sequence of the last exon of DMR-N9, and all of the DM kinase gene. The relative positions of the discovered 15 exons and intervening introns and the noted "B" and "E" endonuclease restriction sites are noted. The complete 13747 bp gene sequence of Figure 5 is set in the following Table I of the Tables section. In comparing to Figure 5, the gene maps within a 14 kb genomic region contained in five contiguous *Bam*HI fragments. The positions of relevant restriction sites are given: B, *Bam*HI; E, *Eco*RI*.* The orientation of the gene is from telomere (TEL) to centromere (CEN). The exons are depicted as boxes and exons 1, 2, 9 and 15 are numbered. Exons 2-8 (black boxes) encode the protein kinase domains, exons 9-12 (strippled boxes) encode the α-helical (coiled coil) domains, and exon 15 (striped box) encodes the hydrophobic, possibly transmembranous domain and contains the CTG repeat. It is understood that various equivalents of the sequence of Table I are included in this invention so that various substitutions in non-functional portions of the gene may be made and thereby encompassed by the sequence of Table I. It is also understood that various single base pair substitutions may be made which encode for the same amino acid of the corresponding codon are also included within the invention. As well, substitutions of base pairs encoding for different amino acids of a functionally equivalent protein variance are included within the invention.

In order to ascertain the putative translational start site of the DM kinase gene, we did a comparative analysis of the sequences from the 5' region of the murine and human versions of the gene.

At the 3' end of the sequence there is a strong sequence homology between the murine and human species. In the 5' region of the sequence, the sequence homology, though present, was found to be scattered with sequence gaps and base deletions. Due to the loss of sequence homology and the known proximity of the DMR-N9 gene (17), the putative translational start codon was identified, based upon the maintenance of the open reading frame for cDNAs previously identified in both species. There are two transcriptional start sites beginning at the 1394 and 1414 position of Table I. In the human, the transcriptional start site of the DM kinase gene may in fact be 5' to an unusual stretch of 24 adenines located approximately 550 bp proximal to the start codon. This region is located at 1516 to 1539 positions of Table I. The predicted full length mRNA of the human gene would be approximately 3.4 kb. The predicted translational product of 629 amino acids, for the longest cDNA for the gene, has a molecular weight of 69,370 daltons. The amino acid sequence of this translational product is provided by Table V and includes the corresponding DNA sequence. The translational start site in Table V corresponds to position 2170 to 2172 in Table I. The transcriptional start site is at position 1 in Table V and position 1394 in Table I.

The precise positions of intron-exon boundaries of Figure 5 were determined by comparison of the genomic and cDNA sequences. Their positions are set out in Table II where the fifteen exons vary in size from 47 bp to 936 bp. The base pair positions of Table II do not correspond with those of Table I. The first Exon on Table II begins at position 1394 of Table I. The human gene has from 2331 bp (the first intron) to 76 bp (the seventh intron). The highly conserved kinase domain is encoded by exons 2 through 8. A region showing significant homology to the α-helical (coiled-coil) domains of myofibrillor and filamentous proteins is encoded by exons 9 through 12, with the strongest homology being contained in exons 11 and 12. Exon 15, the last exon, contains a relatively short region encoding for a hydrophobic, possibly transmembranous domain, and also contains the CTG repeat in the 3' untranslated region. As a consequence of a shift in the open reading frame, an earlier termination condon (5bp after the beginning of exon 15) is used. This results in the loss of the hydrophobic region from the predicted 629 amino acid sequence. The predicted translational product of this isoform has 535 amino acids with molecular weight of 59,790 Daltons.

In the course of sequencing the human gene, numerous sequence polymorphisms were identified within introns and noncoding regions of the gene. The polymorphisms are set out in Table III. The majority of these sequence polymorphisms are due to a single base change.

Several polymorphisms within the human DM kinase gene have been identified and PCR assays to detect two of these are described here. These polymorphisms will be very useful in studying the linkage disequilibrium patterns of these markers with respect to the DM mutation.

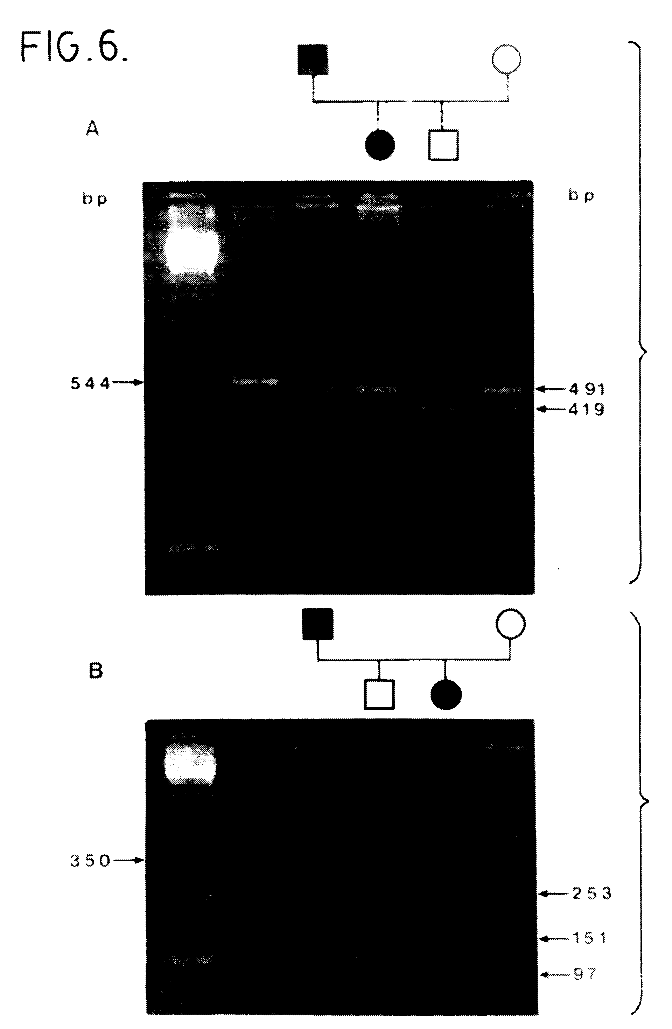

We have developed PCR based assays, in particular for those polymorphisms which have an altered restriction enzyme recognition sequence and which are amenable to simple detection. For the results as shown in Figures 6A and 6B, PCR based assays were used to detect two sequence polymorphisms in the DM kinase gene. In each of figures, A and B, the first lane contains the 123 bp DNA size marker, the next lane contains the uncut PCR amplified product and the remaining lanes contain the digested products from a representative DM pedigree. The sizes of the uncut, and digested products are indicated. A: HhaI Polymorphism. B: HinfI Polymorphism. The larger of two alleles for the HhaI polymorphism (Figure 6A) located in the fifth intron, has an allelic frequency of 0.55. Of the two alleles detected by the HinfI polymorphism (Figure 6B) located in the ninth intron, the larger allele has an allelic frequency of 0.47. Also, a 1 kb insertion/deletion polymorphism, previously noted to be in total linkage disequilibrium with the DM mutation (18, 19, 20), has been characterized and a PCR based assay to detect this flanking marker has been developed (21). The larger of these two alleles, on which the DM mutation always occurs, is found in 53% of normal chromosomes. This polymorphism is contained within the region of the *Alu* repeats that precede exon 9. We have utilized all of these polymorphisms, as the primary tools in documenting a rare gene conversion event which resulted in the loss of the DM mutation in an offspring who inherited the DM chromosome from her affected father (22). The polymorphisms are of significant value in studying linkage disequilibrium patterns in the region of the DM mutation, and can assist in determining the ancestry of the DM mutation in various populations.

The procedure utilized for the gene sequence and detection of polymorphism is as follows. The isolation and detection of chromosome 19q13 cosmid and yeast artificial chromosome (YAC) clones, as well as the characterization of cDNA clones, have been detailed elsewhere (17, 16). Restriction maps of the relevant regions of cosmids Ya100263 and F18894, as well as mouse genomic DNA (17), were generated after hybridization with the cDNA clones. Appropriate subclones of mouse and human genomic DNA were generated for sequence analysis. As well, a shotgun cloning strategy was used to generate clones from cosmid Ya100263. Also, PCR amplified products of human genomic DNA were directly sequenced to confirm much of the sequence generated from the clones. Sequencing strategies included the direct sequencing of subclones with vector primers, sequencing with synthesized oligonucleotides, and sequencing of a graded series of clones with increasing deletions from the 5' end, generated by exonuclease III treatment. Sequencing was performed using standard protocols with ³⁵S and ³²P labelled dNTPs. In addition, sequencing was performed with an ABI 373A automated sequencer using either fluorescent dye labelled primers, or dideoxytermination sequencing reactions with fluorescent dye labelled oligonucleotides. Assembly analysis and alignment of DNA sequences was done with the IG-SUITE 5.35 package (Intelligenetics). David Ghosh's Transcription Factor Database (Rel. 4.5) (23) was scanned using SINGAL SCAN 3.0 (24). As well, the sequence alignment was done using the GAP program in the GCG package (25).

HhaI polymorphism. Genomic DNA (2µg) was PCR amplified with 50 ng each of primers 445 (5' GACCTGCTGACACTGCTGAGC 3') and primer 472 (5' GTGCCTTCCATCCCTCATCAG 3') by a standard protocol and 30 cycles of amplification at 94°C for 1 min., 60°C for 1.5 min., and 72°C for 1.5 min. Amplified products were digested with HhaI for one hour, and subsequently separated by electrophoresis, using 1x TBE buffer, in 1% agarose, 1% NuSieve gels containing ethidium bromide.

HinfI polymorphism. Genomic DNA (2µG) was PCR amplified with 50 ng each of primers 458 (5' CTGCAGAAGGTTTAGAAAGAGC 3') and primer 424 (5' CATCCTGTGGGGACACCGAGG 3') by a standard protocol and 10 cycles of amplification at 94°C for 1 min., 60°C for 1.5 min., and 72°C for 1.5 min. followed by 25 cycles of amplification at 94°C for 1 min., 55°C for 1.5 min., and 72°C for 1.5 min. Amplified products were digested with HinfI for one hour, and subsequently separated by electrophoresis, using 1x TBE buffer, in 1% agarose, 2% NuSieve gels containing ethidium bromide.

To gain a better understanding of the absolute linkage disequilibrium of the CTG repeats and DM and the nature of the insertion/deletion 1-kb element, the 10-kb EcoRI genomic fragment within the DM kinase gene was sequenced and a PCR assay performed to detect this polymorphism as described in the above procedure. Various fragments derived from cosmids F18894 and Ya100263 (25) were cloned into pUC18 or pBluescript vectors. Dideoxy chain termination sequencing reactions were performed with vector primers and synthesized primers, using fluorescent dye-labelled dideoxynucleotides, and subsequently run on an ABI 373A automated sequencer. We also used synthesized primers to generate PCR products containing the insertion element from cosmid F18894. These products were sequenced directly or after cloning the PCR products into an appropriate vector. In view of the various polymorphisms which can be detected by this invention and their utility in deterring susceptibility to DM, we have summarized in Table IV the various preferred primers for use in the PCR amplification process.

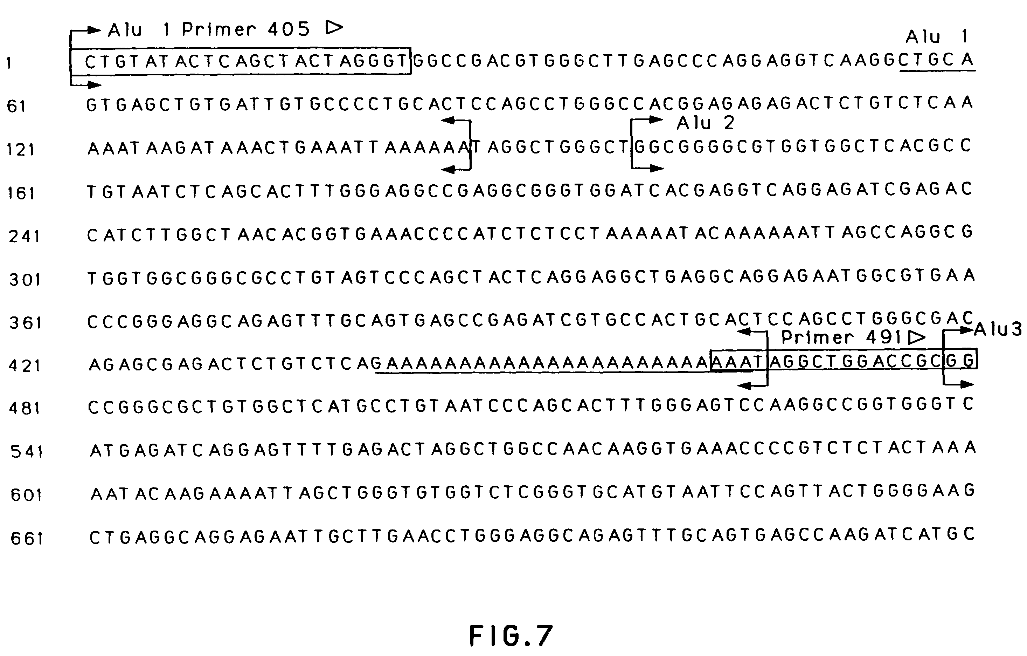

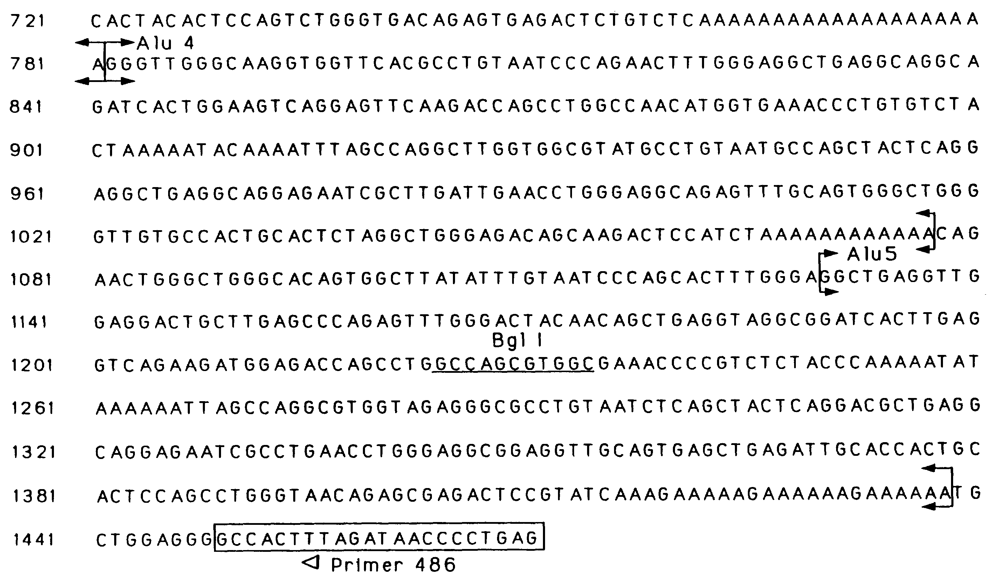

The sequence of this 10 kb region consists of five consecutive *Alu* repeats, all in the same orientation (Mahadevan et al., "Characterization and Polymerase Chain Reaction (PCR) Detection of an *Alu* Deletion Polymorphism in Total Linkage Disequilibrium with Myotonic Dystrophy", *Genomics* 15, 000-000 (1993). An *Alu* repeat is a repetitive 300 base pair sequence which is found throughout the entire human genome. This particular sequence is frequently deleted or inserted in a gene to produce a polymorphism. The *Alu* sequences begin 132 bp centromeric to the region of the probe pGB2.6 (26). Figure 7 depicts DNA sequence of the region containing the deletion polymorphism. Thus, this sequence is somewhat modified as compared to Table I. The region encompasses five *Alu* repeats. The five *Alu* repeats are slight variations of the basic 300 base pair sequence. The depicted sequence of 1470 nucleotides begins in the middle of the first *Alu* repeat and ends distal to the fifth *Alu* repeat. The *Alu* repeats are numbered and their boundaries are bracketed. The restriction sites for the enzymes *Pst*I and *BgI*I*,* previously cited as the boundaries of the insertion/deletion event are underlined. The sequences of the primers used in the PCR assay are boxed and numbered, with arrows indicating the orientation of the primer. The shaded sequence corresponds to the DNA segment involved in the deletion event. The sequence of the PCR product amplified by the assay for this polymorphism of Figure 7, beginning in the middle of the first *Alu* repeat and ending distal to the fifth *Alu* repeat. The second *Alu* repeat is flanked at its 5' and 3' ends by direct repeats. This is usually observed with transposed *Alu* elements (27). There are no similar direct repeats flanking any of the other *Alu* repeats. A comparison of the sequence derived from cosmid F18894 (i.e., the large allele) with the sequence cosmid Ya100263 (the smaller allele) suggests that the smaller allele is a consequence of a "ta" deletion event that resulted in the loss of an equivalent of three *Alu* repeats. The presence of direct repeats flanking the second *Alu* sequence in the larger allele, and the absence of the 3' end of this second *Alu* in the smaller allele, suggest that the insertion of *Alu* repeats into the larger allele predates the deletion event. The proposed deletion event encompasses a region beginning in the left monomer of the second *Alu* repeat and ending in the corresponding region of the left monomer of the fifth *Alu* repeat. This deletion results in a smaller allele in which the remaining *Alu* sequence is contiguous. The boundaries of this proposed deleted segment are not flanked by direct repeats and thus it is less likely that the larger allele arose as a result of an insertion into the smaller allele.

Using primers derived from the sequence, we have developed a PCR-based assay to detect this *Alu* deletion polymorphism. Initial attempts at PCR amplification with flanking primers (405 and 486) flanking the deleted segment resulted in preferential amplification of the smaller allele in heterozygotes.

We have modified the assay by including a third primer (491), derived from the sequence of the deleted fragment. As a consequence, a more equal amplification of the two alleles was observed. Because primer 491 is specific for the deleted fragment, the PCR amplification of even a faint upper band is indicative of the presence of the larger allele. This allele is in complete linkage disequilibrium with DM. This PCR assay has significant diagnostic value as it detects a polymorphism that is the closest, documented, distal marker to the DM mutation (26).

Given the knowledge of the major mutation as disclosed herein, carrier screening and prenatal diagnosis can be carried out as follows.

One major application of the DNA sequence information of the DM gene is in the area of genetic testing, and prenatal diagnosis. The demonstration of an increased CTG trinucleotide repeat length in individuals with severe congenital DM provides firm molecular evidence for genetic anticipation (Tsilfidis et al., "Correlation Between CTG Trinucleotide Repeat Length and Frequency of Severe Congenital Myotonic Dystrophy", *Nature Genetics,* Vol. 1 pp. 192-194 (1992)). The majority of DM mothers with noncongenital DM children manifest E0-E1 stage CTG repeat lengths while mothers of congenital DM patients belong to E1-E3 classifications. Arbitrarily we have applied the following values to the E0, E1, E2, E3 and E4. E0 indicates no expansion visible on South blotting; E1 is an expansion of 0 to 1.5 kb (0-500 CTG repeats); E2 is an expansion of 1.5 kb to 3.0 kb (up to 1000 CTG repeats); E3 is an expansion of 3.0 to 4.5 kb (up to 1500 repeats); and E4 is an expansion greater than 4.5 kb (up to 2000 repeats). Furthermore, and in contrast to the situation with noncongenital DM offspring, all congenital DM children have CTG repeats which are demonstratably larger than those of their affected parent. In noncongenital parent/child pairings, the degree of intergenerational CTG amplification is not dependent on which parent (mother or father) contributes the DM allele. CTG amplification in DM is not gender dependent but congenital DM very much is, resulting exclusively from maternal transmission of the DM mutation. These results were derived as follows. CTG amplification was examined in 272 DM and 140 non-DM individuals. Included in the myotonic group were 22 congenital DM individuals and their mothers and 230 randomly sampled individuals with noncongenital myotonic dystrophy. The diagnostic criteria for congenital DM were: (a) Marked hypotonia at birth. (b) Respiratory failure necessitating ventilation at birth. (c) Absence of an underlying condition which would lead to hypotonia or respiratory failure (for example, infection). (d) A diagnosis of DM in the infant's mother. Using these stringent criteria to define congenital DM, it is likely that infants with milder forms of congenital DM exist in our population but have not been ascertained. Nonetheless, we believe that our ascertainment of the more clinically relevant severe congenital DM approaches 100%. Genomic DNA (5µg) was digested with EcoRI (New England Biolabs), electrophoresed on 0.8% agarose gels and transferred onto nylon membranes (Hybond). Southern blots were probed with the 2.2 kb BamHI/EcoRI subclone of probe pGB2.6. The genomic probe pGB2.6 maps to the 10 kb EcoRI fragment containing the variable length polymorphism. Blots were washed at 0.2 x SSC, 0.1% SDS, 65 C and exposed to Kodak XAR film for 1-4 days.

The age of onset of DM has been correlated with the number of CTG trinucleotide repeats (Hunter et al., "The Correlation of Age of Onset with CTG Trinucleotide Repeat Amplification Myotonic Dystrophy", *J. Med. Genet*. 29:774-779 (1992). Broad age categories were used to search for correlations between allele expansion and age of onset as follows: congenital, postnatal and < 5 years, 5 to < 15 years, 15 to <25 years, 25 to < 35 years, 35 to < 45 years, 45 to < 60 years, above 60 years, and as clinically unaffected at the time of study. Every attempt was made to correlate this timing by noting the results of previous medical examinations, by examining photographs, and by confirming the information with other family members. Seventeen families were chosen providing a total of 109 DNA proven positive DM subjects.

Southern blot DNA analysis was conducted as described above. In addition polymerase chain reaction amplification of the CTG repeat provided a more accurate assessment of its size. It detects expansion not visible on Southern blots of E0 subjects and was used to rank the size of expansion between family members who had the same E0 to E2 class.

Genomic DNA (1µg) was PCR amplified with primers 406 and 409 using standard protocol of 30 cycles at 94°C for one minute, 60°C for one minute, and 72°C for 1.5 minutes. Amplified DNA was electrophoresed on 1% agarose gels, vacuum blotted onto nylon membrane (Hybond^{Tm, Amersham}), and probed with a labelled (CTG)₁₀ oligonucleotide. Membranes were washed in 6 x SSC, 0.1% SDS at 45°C and exposed to Kodak XAR film for 1.0 to 2.5 hours at -80°C.

Despite a significant overlap between age groups, a trend to earlier age of onset with increasing allele size is readily apparent. This trend is most distinct in the E0 category in which only one of the 23 patients had onset of clinical symptoms before the age of 25 years. The majority of E1 patients had onset between the age of 15 and <15 years. In the E2 class, 40% had onset between 5 and <15 years. There was a broad range of age of onset among the E3 class, but an important 28% had onset under the age of 5 years. Congenital myotonia (Hunter et al., "The Correlation of Age of Onset with CTG Trinucleotide Repeat Amplification in Myotonic Dystrophy", *J*. *Med. Genet.* 29:774-779 (1992)), occurred in 3% of E2 and 5-6% of E3 patients. There were only seven patients with class E4 alleles, with four being congenital cases.

Parents and their children (55 parent/child pairs) were compared for allele size and age of onset. In all 38 pairs where the child showed allele expansion beyond the class seen in the affected parent there was an earlier onset in the child. Furthermore, in 33 of these 37 pairs the child was two or more age groupings earlier in onset than the parent (24 cases) or the parent was asymptomatic at the time of study (nine cases). In 15 of 17 cases where the parent and child were in the same allele size class the child had earlier onset than the parent.

The mother was the gene carrier in 35 cases and the father in 17 cases, when an earlier onset occurred in the child (X²⁼6.2, p < 0.025). Furthermore, in nine of 11 cases where the parent was clinically unaffected at the time of study, it was the father who transmitted the DM gene. In contrast, six of seven children with onset from birth to < 5 years received the gene from their mother. Twenty-nine of 45 sibling pairs were either matched for age of onset and allele class, or the sibling with earlier onset had greater expansion of the allele. In only one of the seven pairs where siblings had apparently similar alleles but an earlier age of onset in one sibling, was the difference more than one age category. The six sibling pairs who differed in allele class but had the same age of onset differed by a single class.

Patients and their families were asked whether the person was considered a superior or average student and whether they had repeated a grade(s) or had required a special education or vocational programme. The distribution of school performance in the E0 class is as would be expected from the general population. However, over 40% of subjects in the E1 category had failed at least one grade, while failures or the need for special/vocational education occurred in most persons with ≥ E2 expansion. The numbers in the E4 category are too small to generalize.

Individuals carrying mutations in the DM gene may be detected at the DNA level with the use of a variety of techniques. The genomic DNA used for the diagnosis may be obtained from body cells, such as those present in peripheral blood, urine, saliva, tissue biopsy, surgical specimen and autopsy material. The DNA may be used directly for detection of specific sequence or may be amplified enzymatically in vitro by using PCR (Saiki et al. *Science* 230:1350-1353, (1985), Saiki et al. *Nature* 324:163-166 (1986)) prior to analysis. RNA or its cDNA form may also be used for the same purpose. Recent reviews of this subject have been presented by Caskey, *Science* 242:229-237 (1989).

The detection of specific DNA sequences may be achieved by methods such as hybridization using specific oligonucleotides (Wallace et al. *Cold Spring Harbour Symp. Quant. Biol.* 51:257-261 (1986)), direct DNA sequencing (Church and Gilbert, *Proc. Nat. Acad. Sci. U.S.A.* 81:1991-1995 (1988)), the use of restriction enzymes (Flavell et al. *Cell* 15:25 (1978), Geever et al. *Proc. Nat. Acad. Sci. U.S.A.* 78:5081 (1981)), discrimination on the basis of electrophoretic mobility in gels with denaturing reagent, (Myers and Maniatis, *Cold Spring Harbour Sym. Quant. Biol.* 51:275-284 (1986)), RNase protection (Myers, R.M., Larin, J., and T. Maniatis *Science* 230:1242 (1985)), chemical cleavage, (Cotton et al *Proc. Nat. Acad. Sci. U.S.A.* 85:4397-4401 (1985)) the ligase-mediated detection procedure, (Landegren et al *Science* 241:1077 (1988)) and the single strand conformation polymorphism (SSCP) technique, (Orita et al *Proc. Natl. Acad. Sci.* 86:2766-2770 (1989)).

Oligonucleotides specific to normal or mutant sequences are chemically synthesized using commercially available machines, labelled radioactivity with isotopes (such as ³²P) or non-radioactively (with tags such as biotin (Ward and Langer et al. *Proc. Nat. Acad. Sci. U.S.A.* 78:6633-6657 (1981)) and hybridized to individual DNA samples immobilized on membranes or other solid supports by dot-blot or transfer from gels after electrophoresis. The presence or absence of these specific sequences are visualized by methods such as autoradiography or fluorometric, (Landegren et al. 1989, *supra*) or colorimetric reactions, (Gabeyehu et al. *Nucleic Acids Research* 15:4513-4534 (1987)).

Sequence differences between normal and mutants may be revealed by routine DNA sequencing methods. Cloned DNA segments may be used as probes to detect specific DNA segments. The sensitivity of this method is greatly enhanced when combined with PCR or other amplification procedures, (Wrinchnik et al. *Nucleic Acids Res.* 15:529-542 (1987)); Wong et al. *Nature* 330:384-386 (1987)); Stoflet et al. *Science* 239:491-494 (1988)). In the latter procedure, a sequencing primer which lies within the amplified sequence is used with double-stranded PCR product or single-stranded template generated by a modified PCR. The sequence determination is performed by conventional procedures with radiolabeled nucleotides or by automated sequencing procedures with fluorescent-tags.

Sequence alterations may occasionally lead to a loss or a gain of a restriction enzyme recognition site which can be revealed by the use of appropriate enzyme digestion followed by conventional gel electrophoresis and/or blot hybridization procedures, such as, Southern Blot *(J. Mol. Biol.* 98:503 (1975)). DNA fragments carrying the site (either normal or mutant) are detected by their reduction in size or increase of corresponding restriction fragment numbers. Genomic DNA samples may also be amplified by PCR prior to treatment with the appropriate restriction enzyme; fragments of different sizes are then visualized under UV light in the presence of ethidium bromide after gel electrophoresis.

Genetic testing based on DNA sequence differences may be achieved by detection of alteration in electrophoretic mobility of DNA fragments in gels with or without denaturing reagent. Small sequence insertions can be visualized by high resolution gel electrophoresis. DNA fragments of different sequence compositions may be distinguished on denaturing formamide gradient gel in which the mobilities of different DNA fragments are retarded in the gel at different positions according to their specific "partial-melting" temperatures, (Myers, *supra*)*.* In addition, sequence alterations, may be detected as changes in the migration pattern of DNA heteroduplexes in non-denaturing gel electrophoresis, (Nagamine et al, *Am J. Hum. Genet,* 45:337-339 (1989)). Alternatively, a method of detecting a mutation comprising a base substitution or other change could be based on differential primer length in a PCR. For example, one invariant primer could be used in addition to a primer specific for a mutation. The PCR products of the normal and mutant genes can then be differentially detected in acrylamide gels.

Sequence changes at specific locations may also be revealed by nuclease protection assays, such as RNase (Myers, *supra)* and S1 protection, (Berk, A. J., and P. A. Sharpe, Proc. *Nat. Acad. Sci. U.S.A.* 75: 1274 (1978)), the chemical cleavage method, (Cotton, *supra*) or the ligase-mediated detection procedure (Landegren, *supra*) or by SSCP analysis (Orita, *supra*)*.*

In addition to conventional gel-electrophoresis and blot-hybridization methods, DNA fragments may also be visualized by methods where the individual DNA samples are not immobilized on membranes. The probe and target sequences may be both in solution or the probe sequence may be immobilized (Saiki et al. *Proc. Nat. Acad. Sci. U.S.A.,* 86:6230-6234 (1989)). A variety of detection methods, such as autoradiography involving radioisotopes, direct detection of radioactive decay (in the presence or absence of scintillant), spectraphotometry involving colorigenic reactions and fluorometry involving fluorogenic reactions, may be used to identify specific individual genotypes.

As discussed above, nearly 70% of DM individuals show distinct expansion of the variable region using Southern Blot analysis of restricted genomic DNA. However, most of the remaining 30% that do not show Southern Blot allelic expansion show some increase in the sizes of the mutant allele using the PCR-based oligonucleotide assay. Only a few of our DM families (2/98) are negative for both types of analysis. An explanation is that these families represent unique mutations of the DM gene that do not involve an increase in the number of CTG repeats.

Occasionally, in DM individuals, a faint higher molecular weight band can be PCR amplified and be detected using ethidium bromide and agarose gel electrophoresis. This occurs only with DNA from DM individuals that show minimal or no visible expansion of the 10 kb *Eco*RI or 9.5 kb *Hind*III DM allele. However, we have determined that these bands produce an intense hybridization signal when bound to the (CTG)₁₀ oligonucleotide (Fig. 4). Selective PCR amplification of these bands followed by direct DNA sequence analysis showed no differences in the DNA sequence flanking the CTG repeat (from primer 407 to primer 409, see Fig. 1) in comparison to normal individuals. Only the number of CTG repeats in the 2 insert appeared to vary between normal and DM alleles, although this was difficult to determine unequivocally because of severe compression artifacts in the sequencing gels, a problem similarly encountered in the DNA sequence analysis of CGG repeat in the fragile X mutation (11). However, our compression problems were typically evident after about 10 trinucleotide repeat units were sequenced from either strand of the mutant allele. This sequencing data, combined with the observation that strong hybridization smears were produced by probing with the (CTG)₁₀ oligonucleotide, clearly establishes that the difference in this region between the DM and normal alleles is due to the variable number of CTG repeats.

Our results show that DM is caused primarily by a mutation that increases the size of a genomic fragment due to the dramatic increase in the amplification of CTG repeat. This is similar to the trinucleotide repeat mutation in Fragile X (10,11) and in X-linked spinal and bulbar muscular atrophy (13). Nearly all DM individuals (98%) in our population display an increased number of CTG repeats which can be detected by a variety of assay techniques as previously discussed. Specifically, Southern Blot analysis of genomic DNA and/or hybridization analysis of the PCR-based assay described can be use. We have demonstrated that these trinucleotide repeats are located within the 3' untranslated region of the last exon of this DM gene. This gene encodes a putative serine/threonine protein kinase, as deduced from the DNA sequence analysis of human (14) and homologous mouse (15) cDNAs.

We have found considerable variability in the number of CTG repeats in normal chromosomes which repeats may range from 5 to 30 repeats. Based on this finding, the variability factor can be used in conjunction with established DNA procedures of identity determination such as would be applied in paternity analysis and forensic applications.

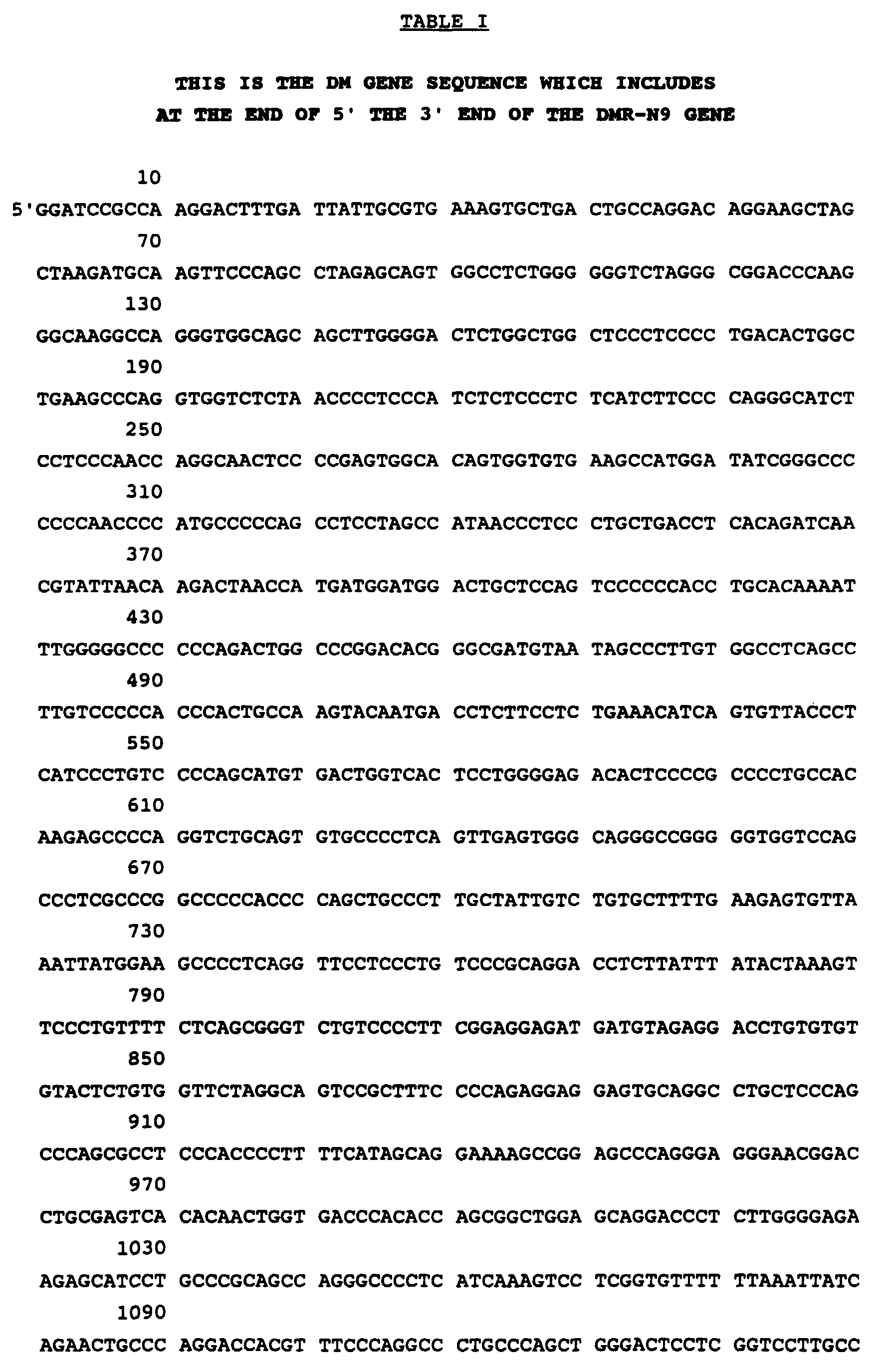

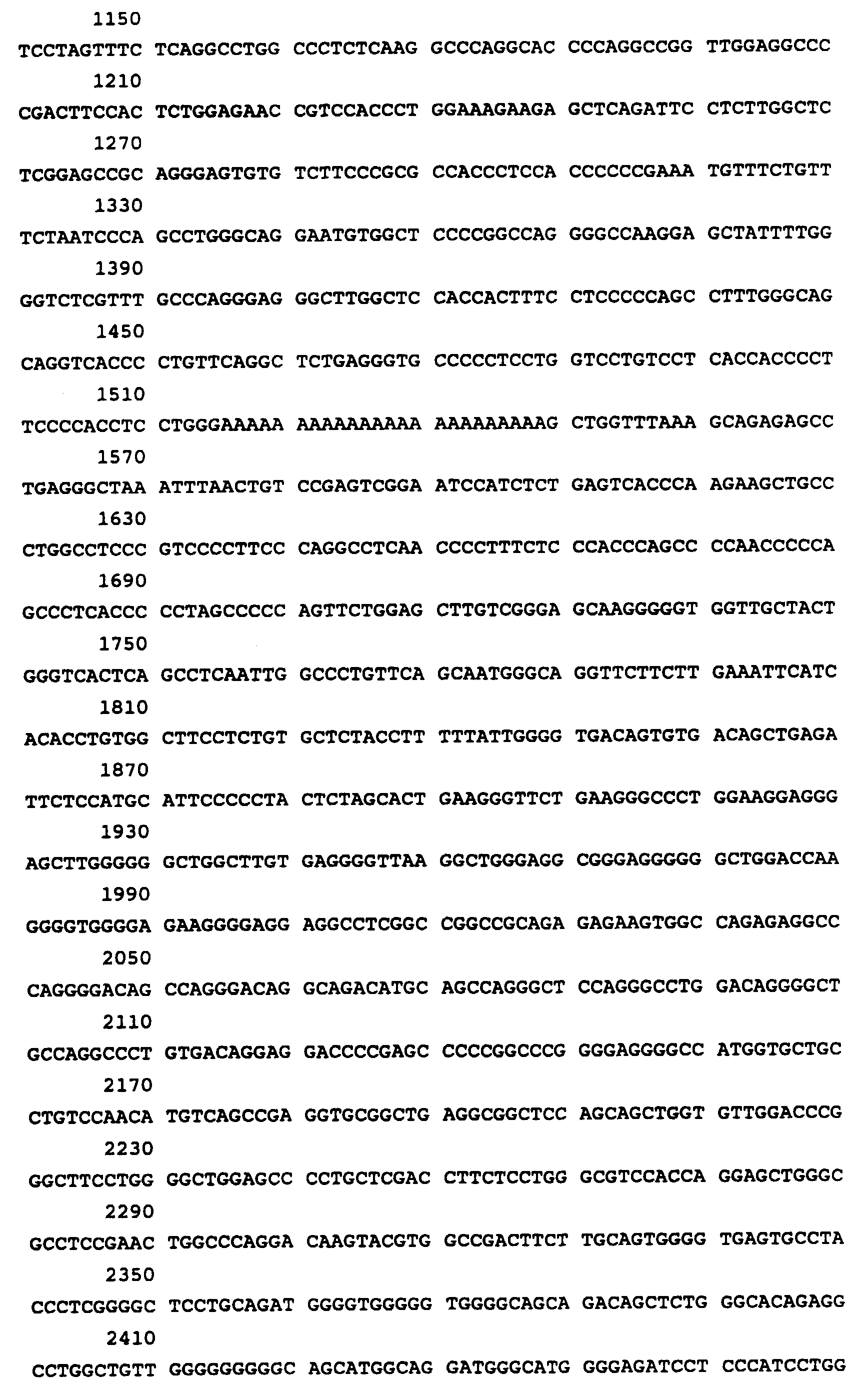

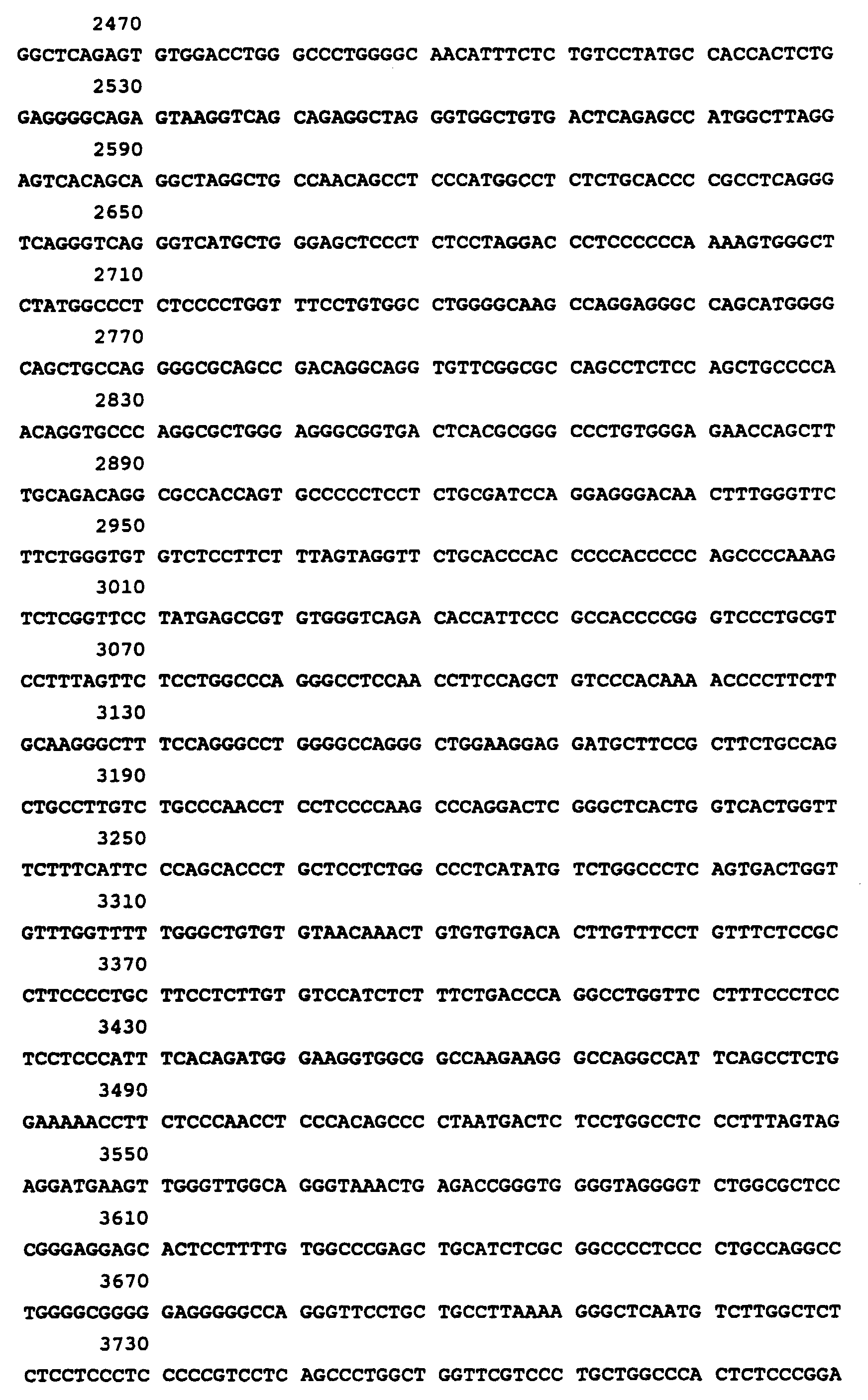

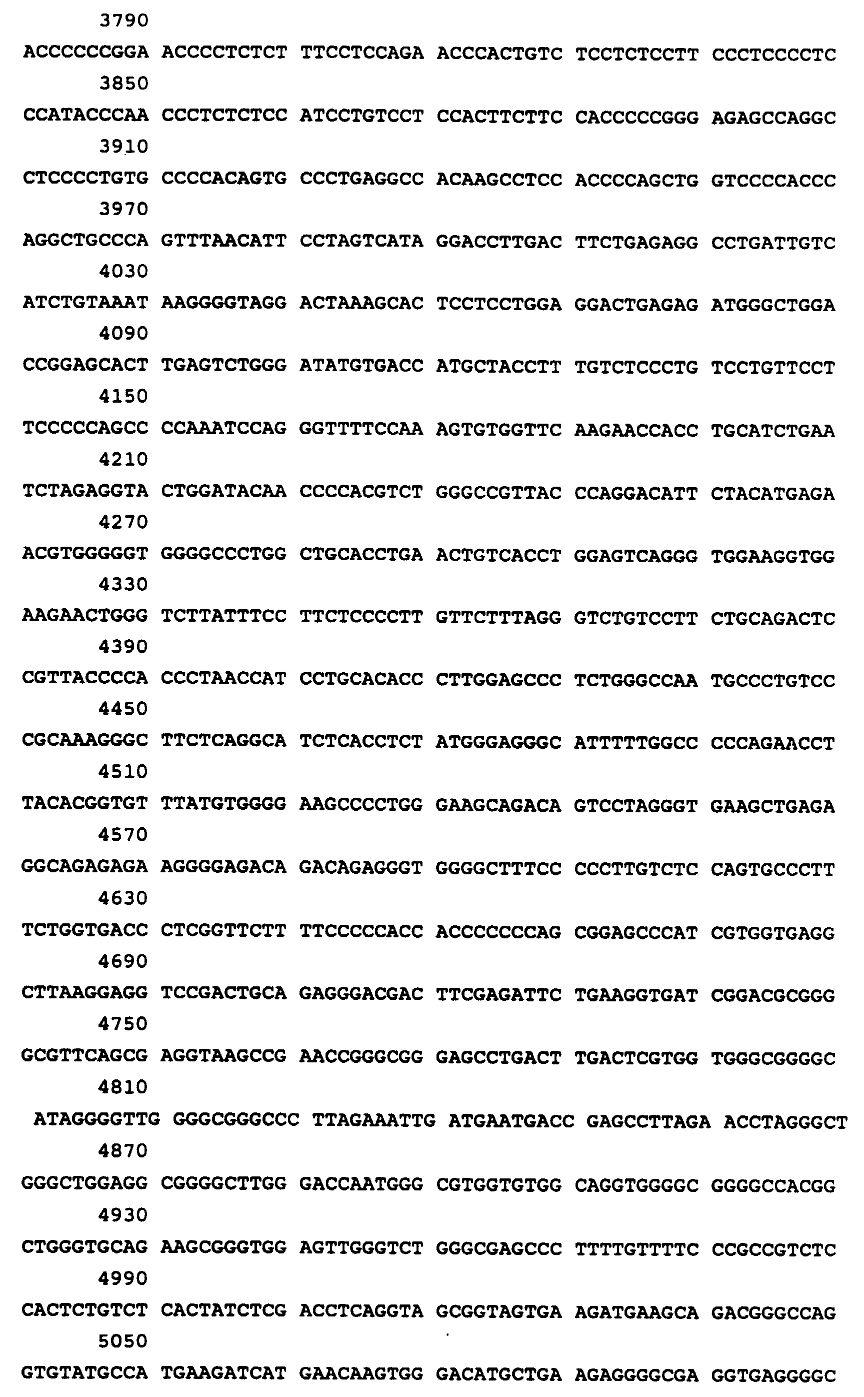

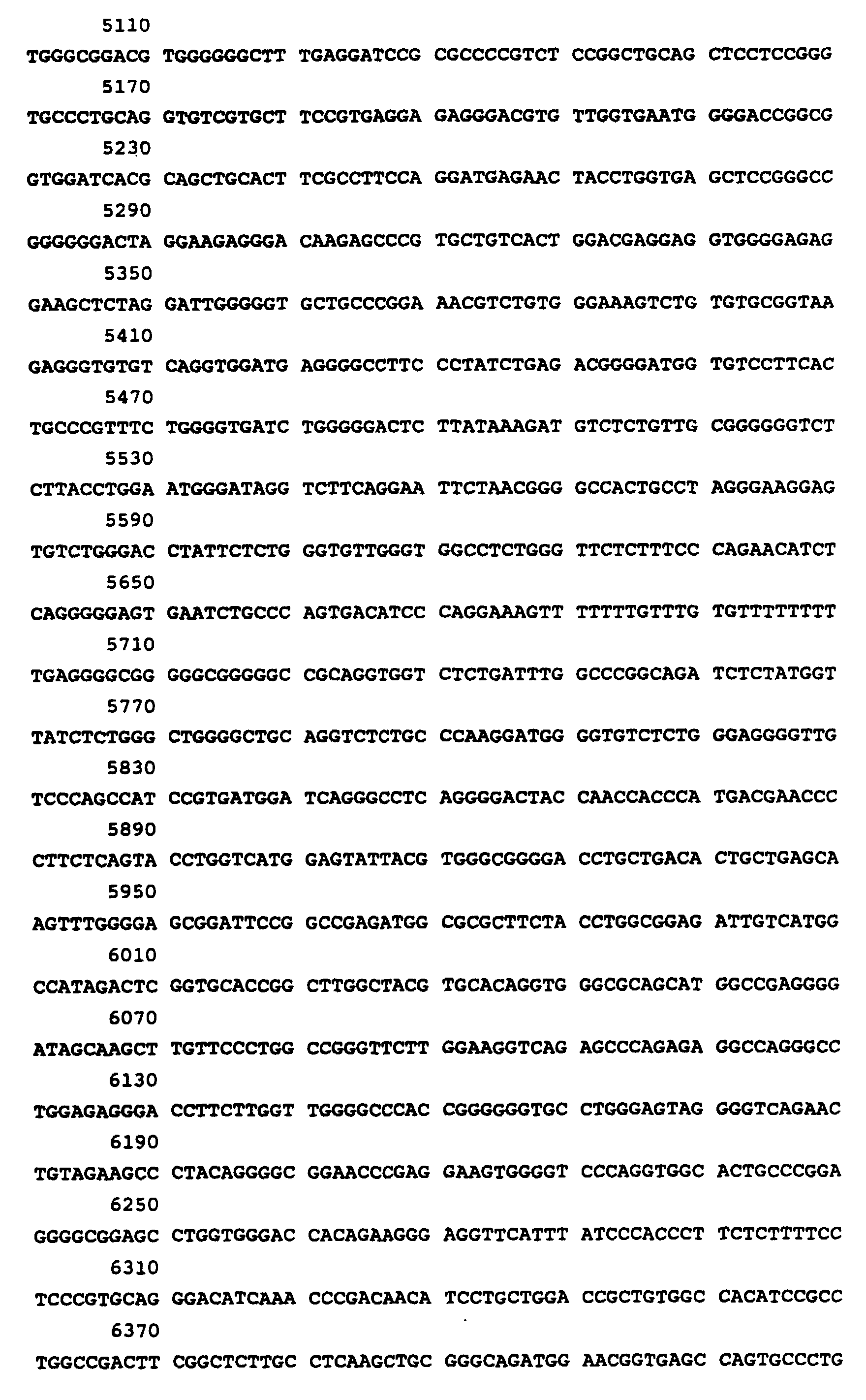

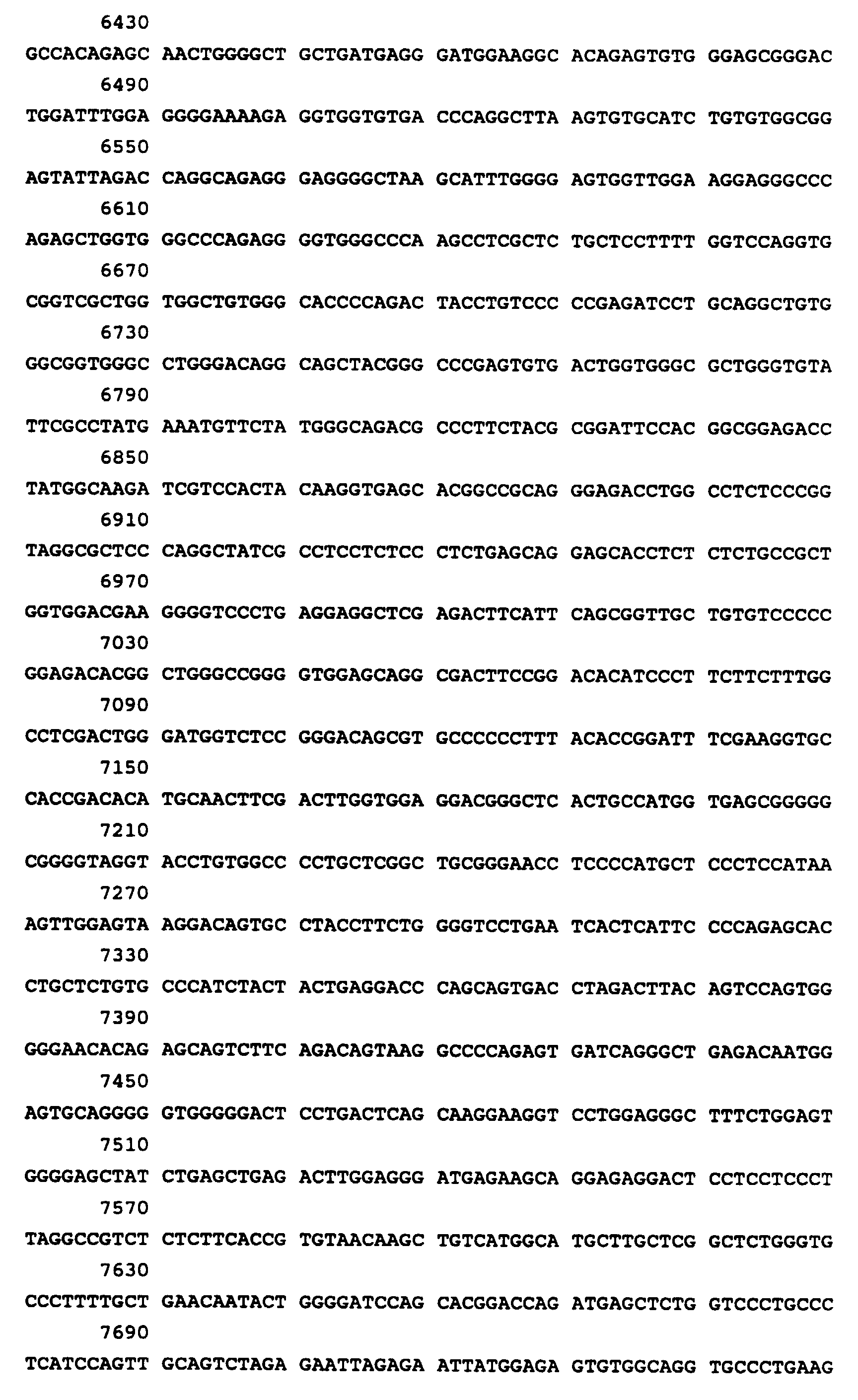

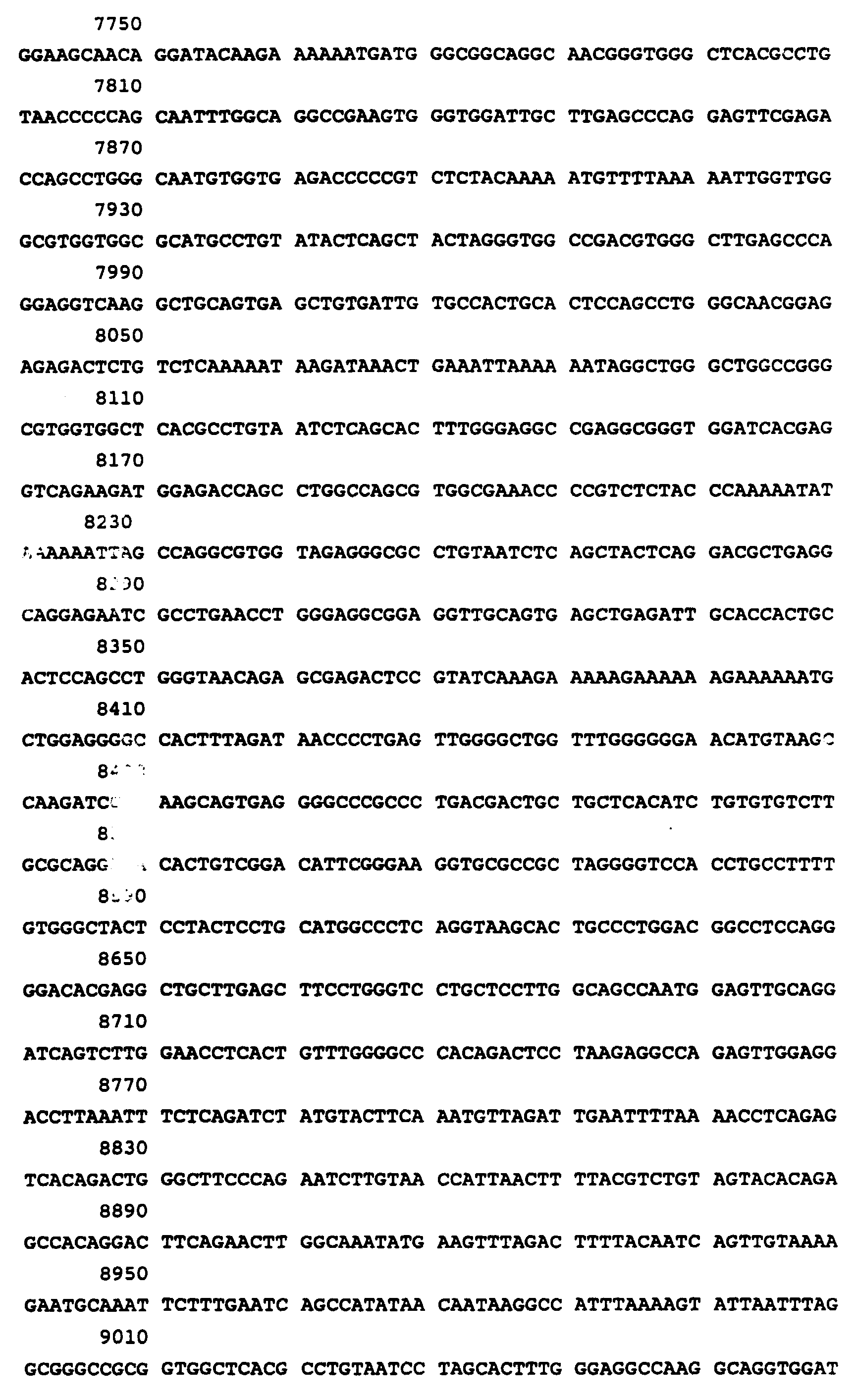

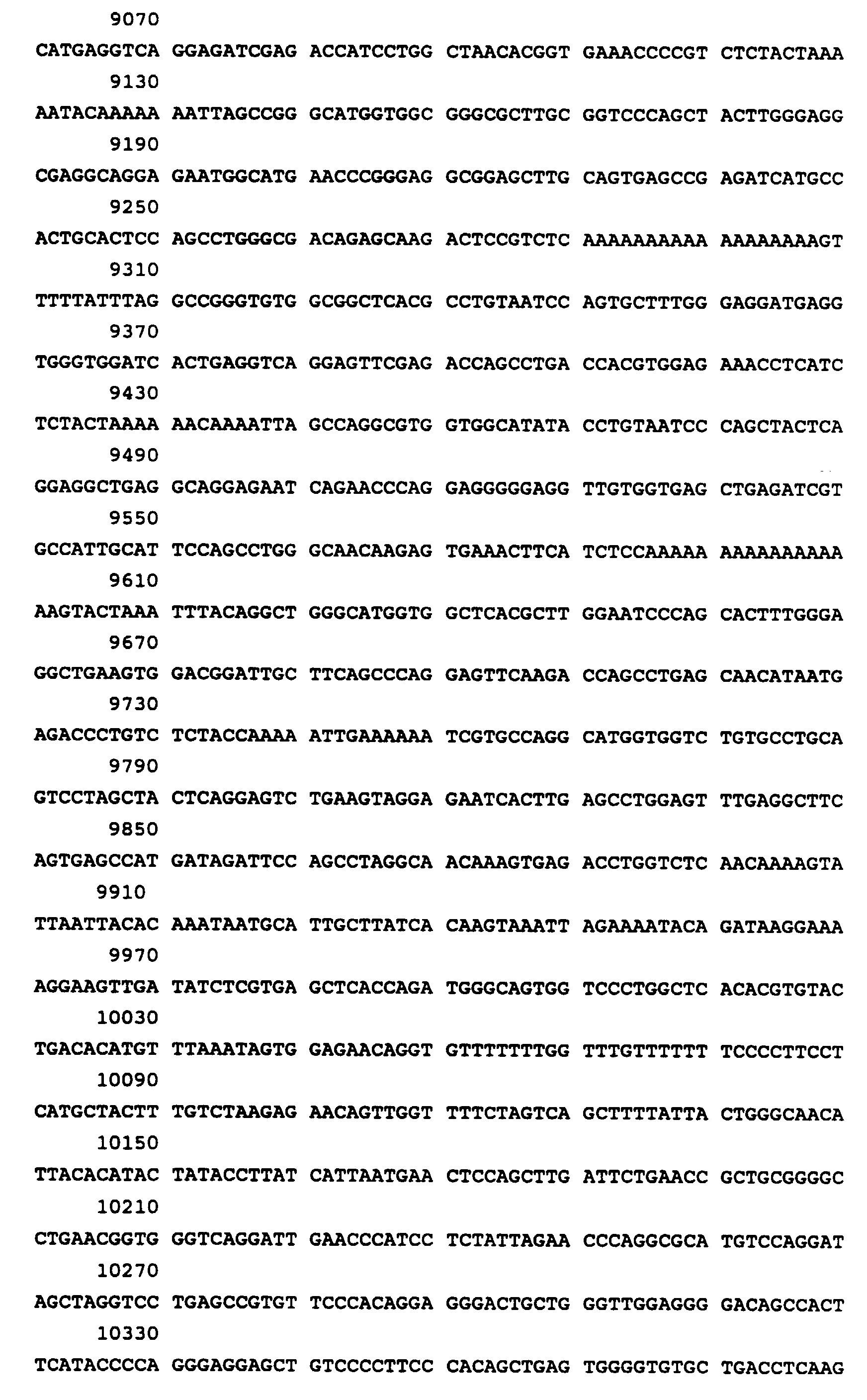

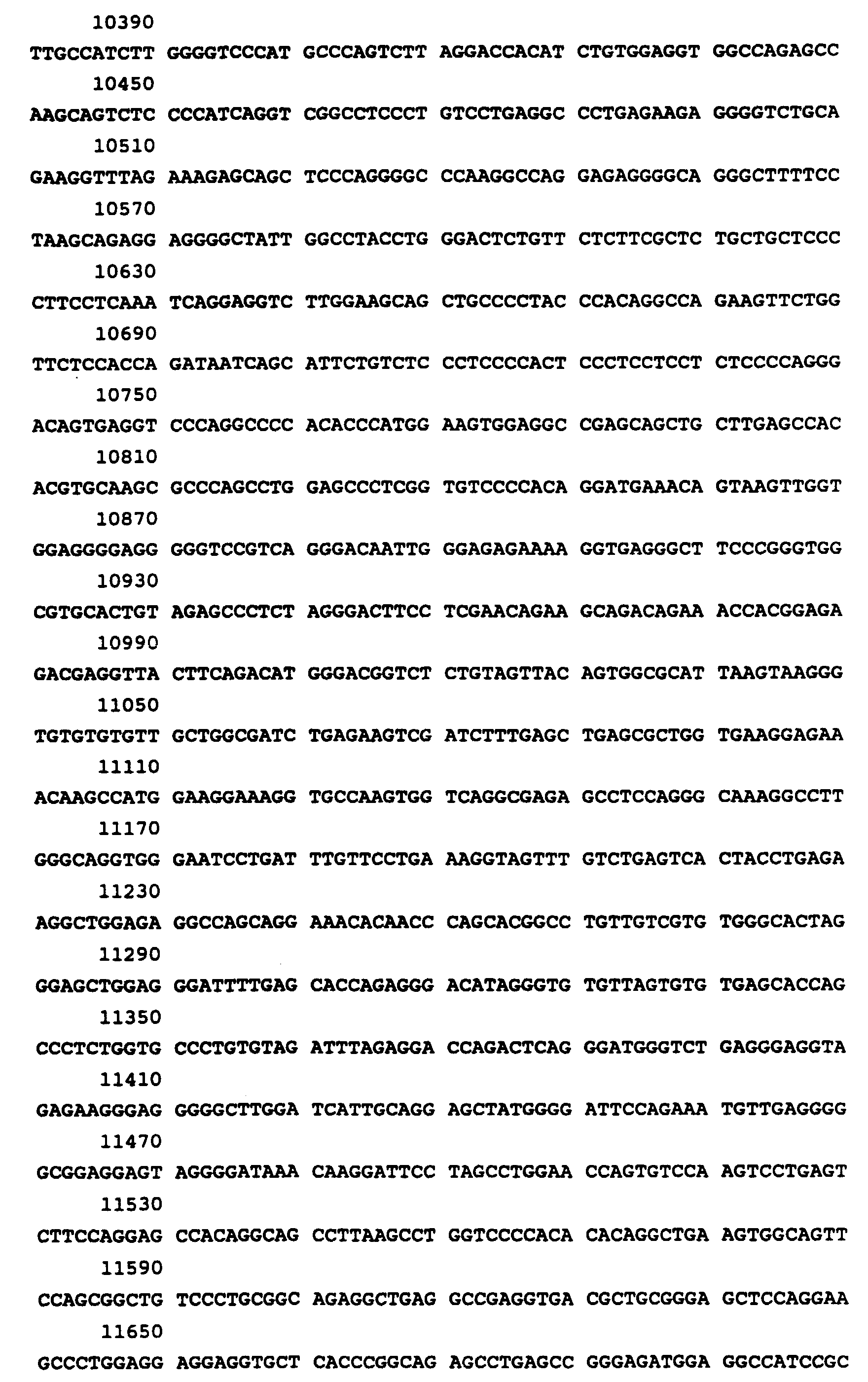

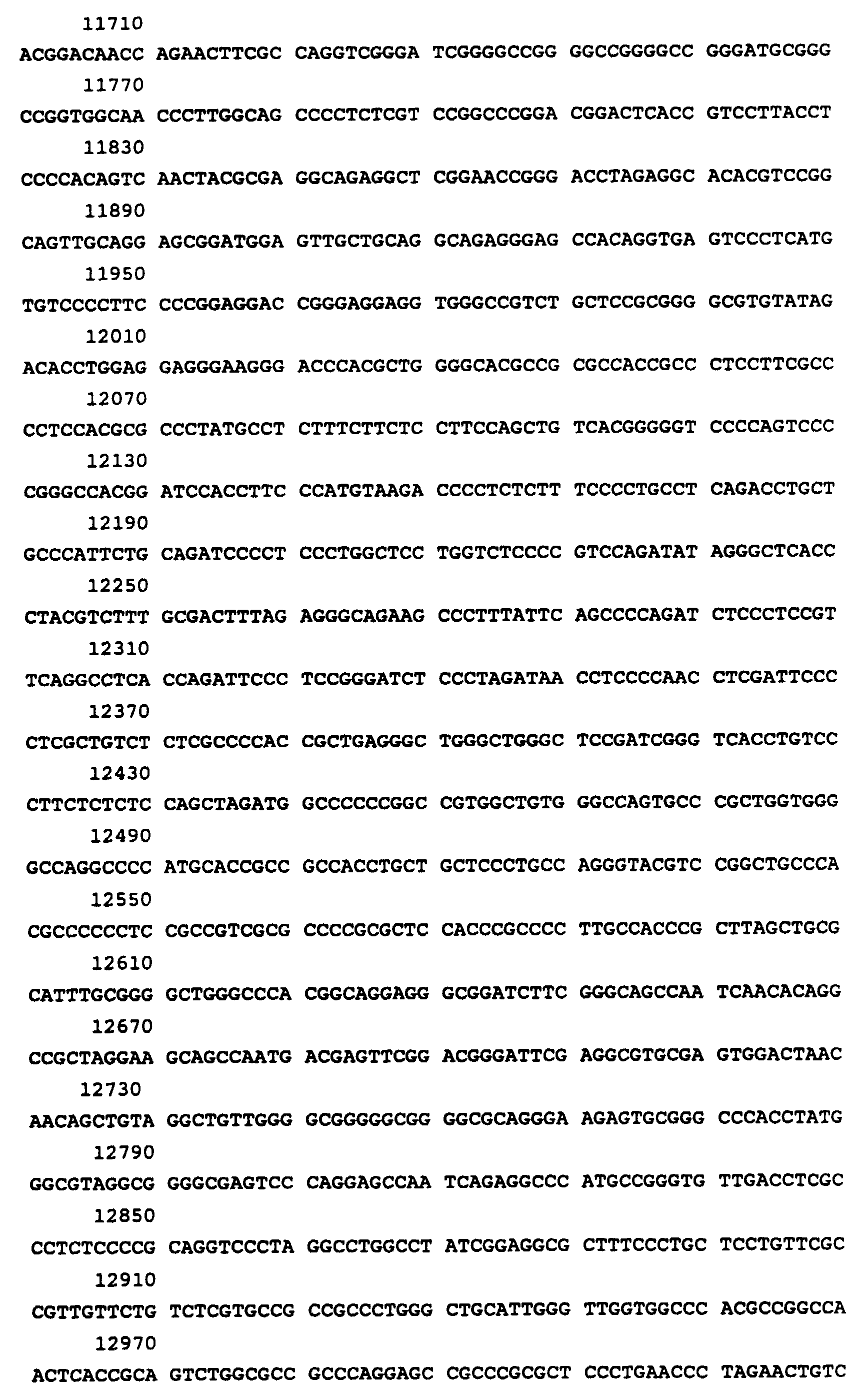

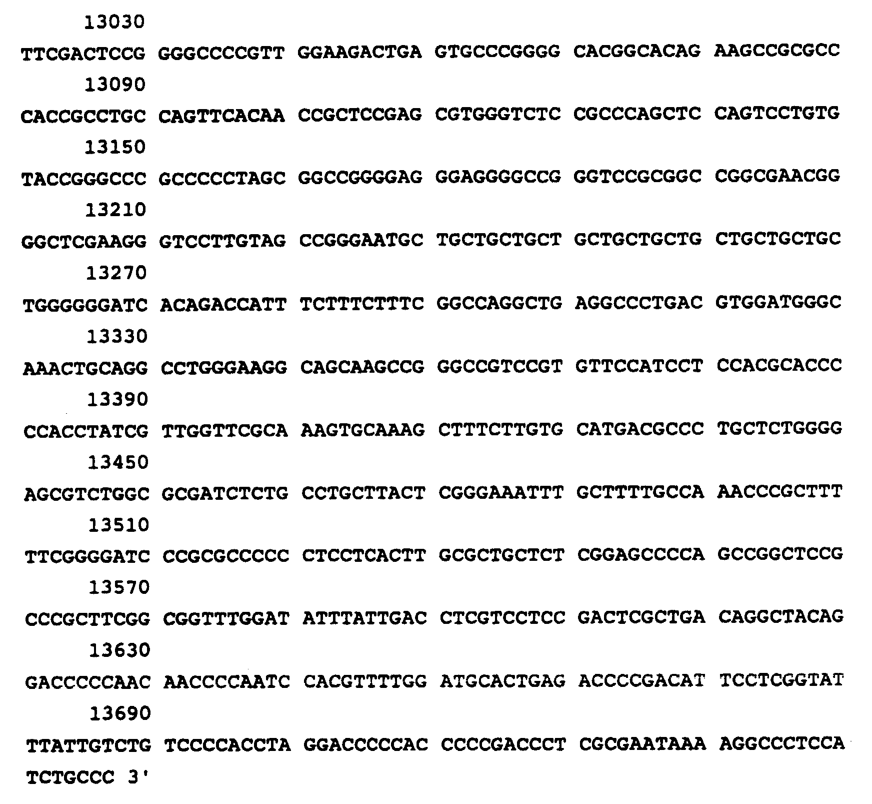

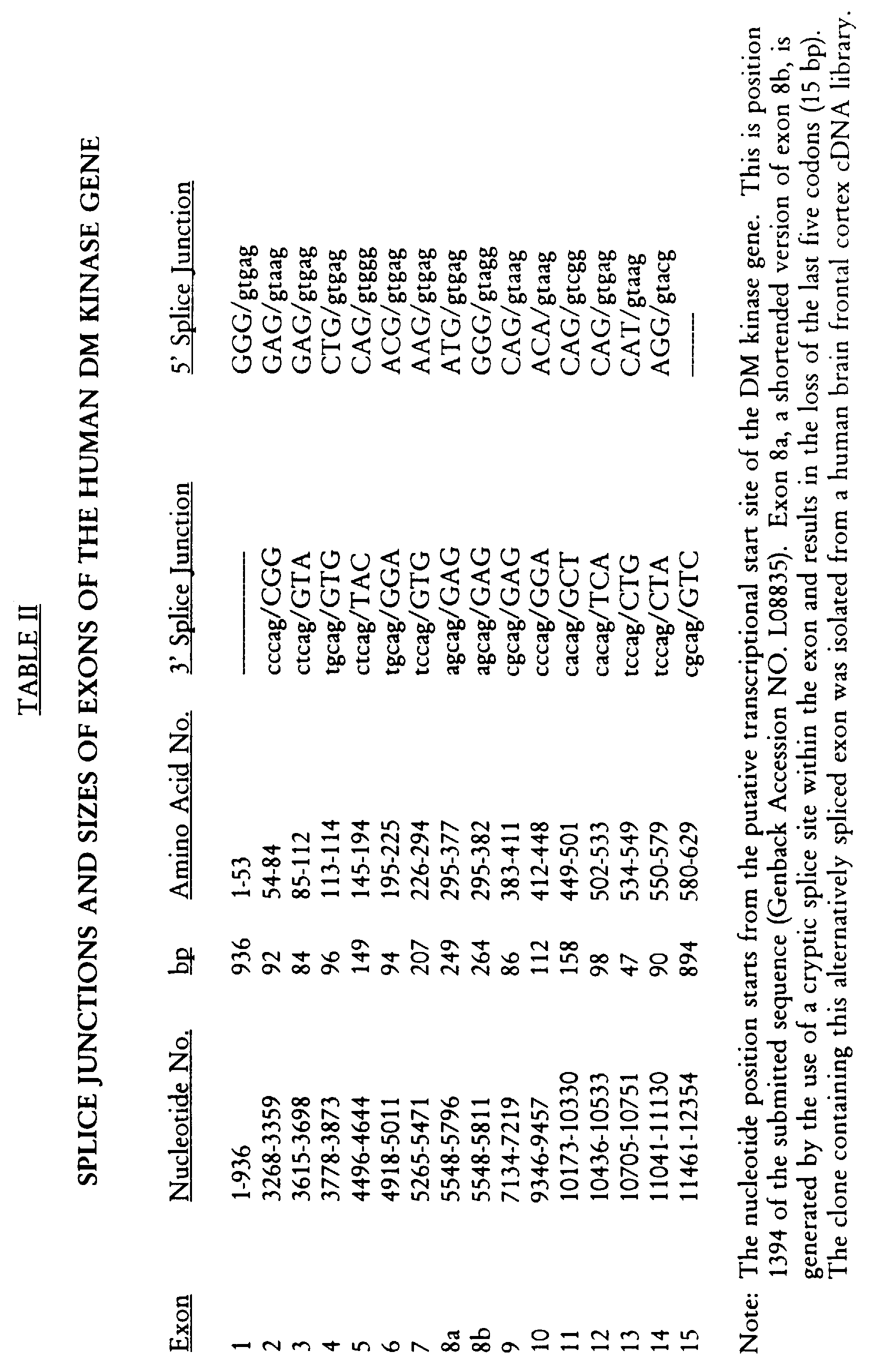

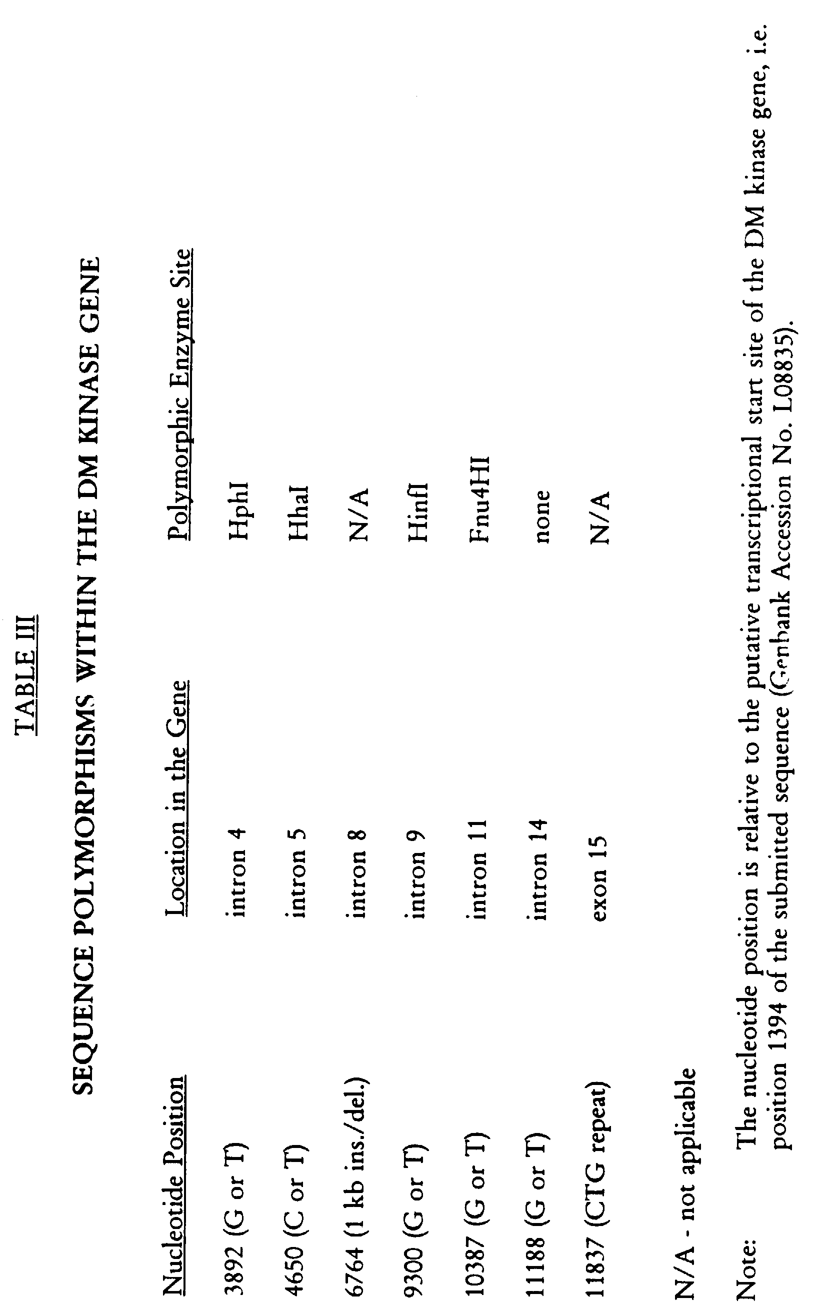

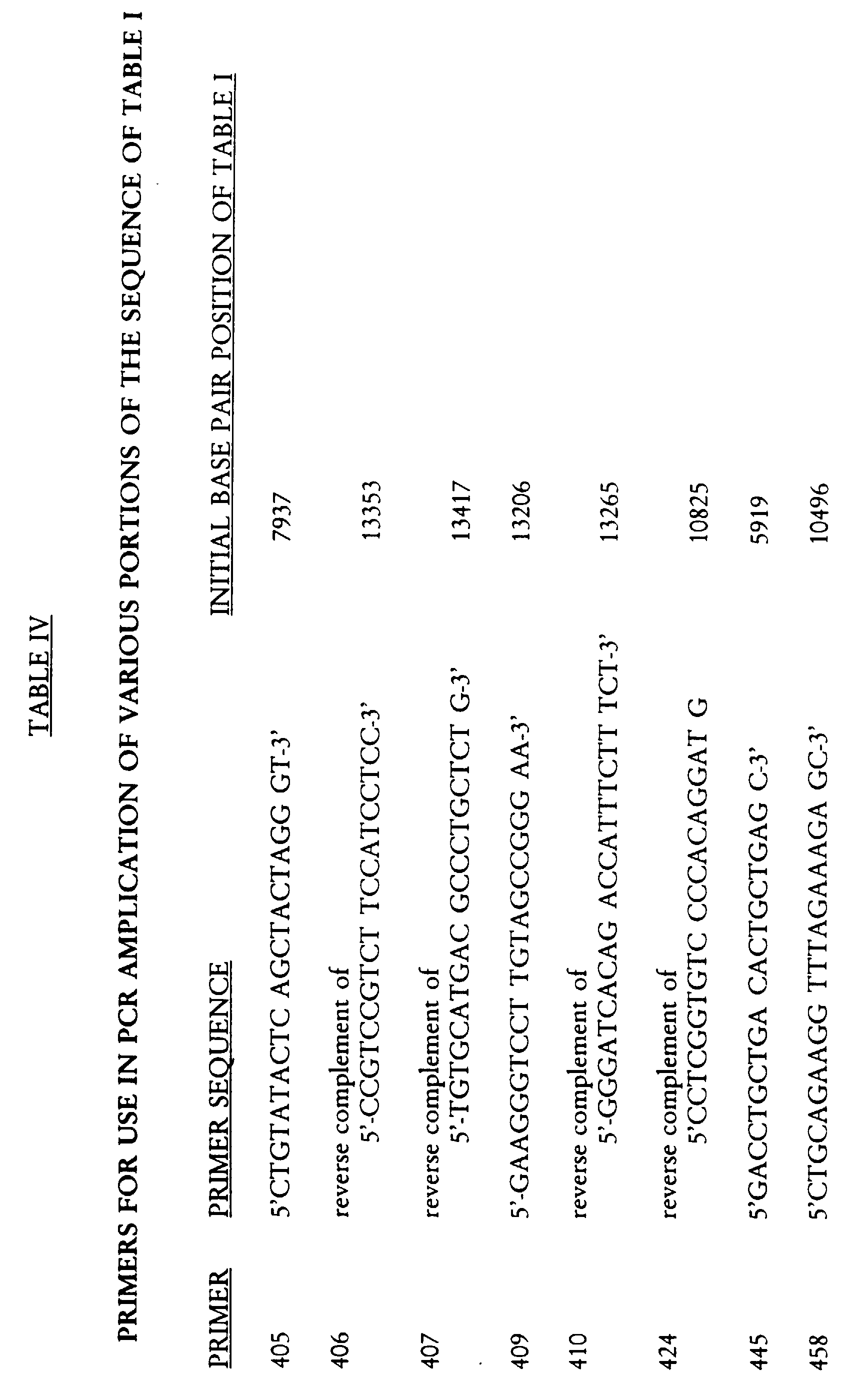

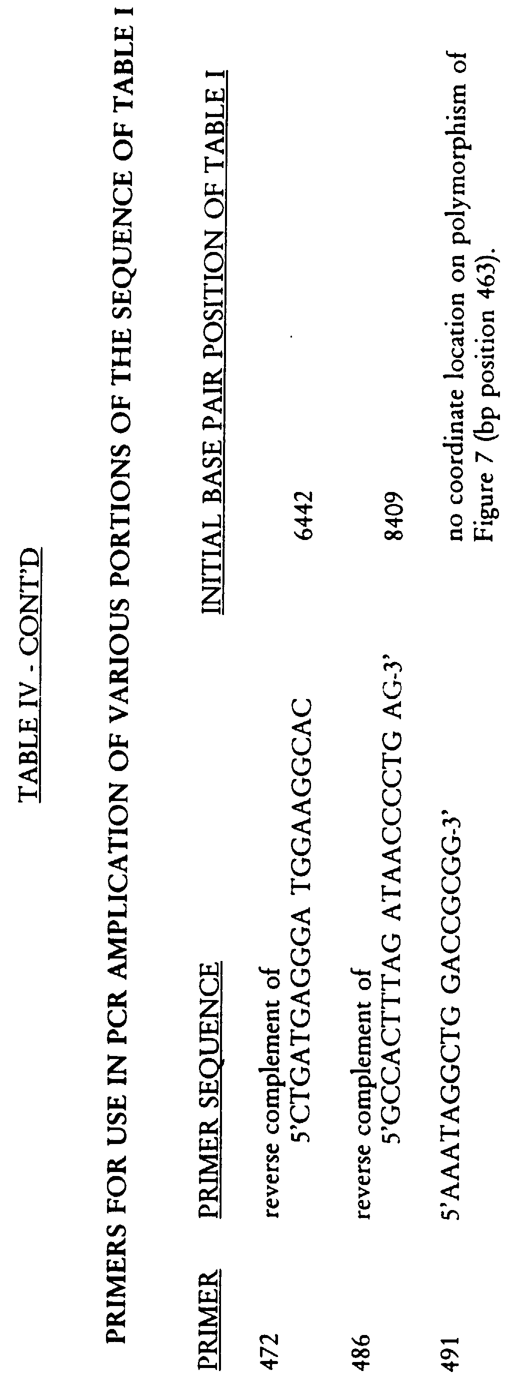

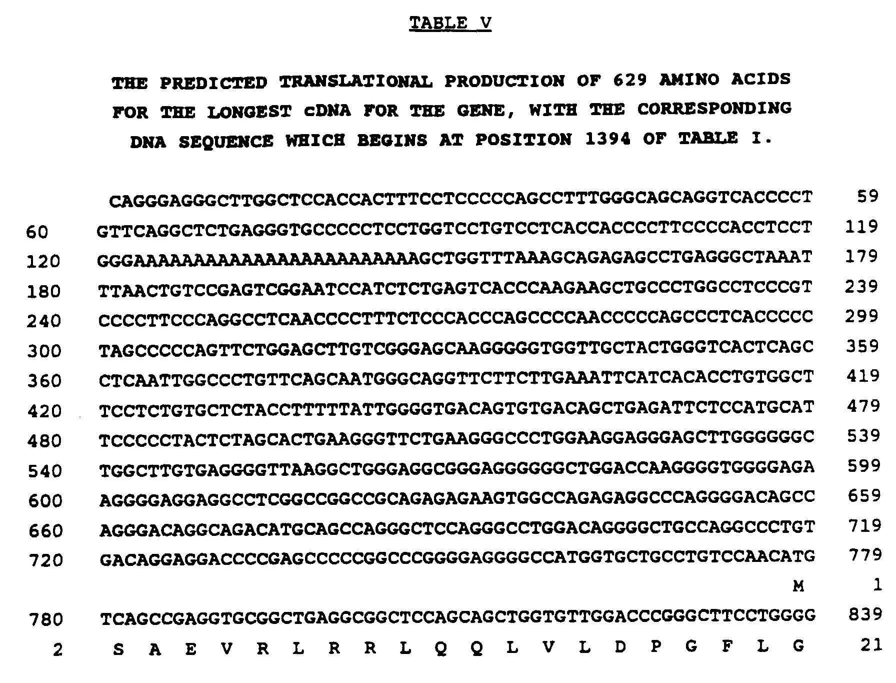

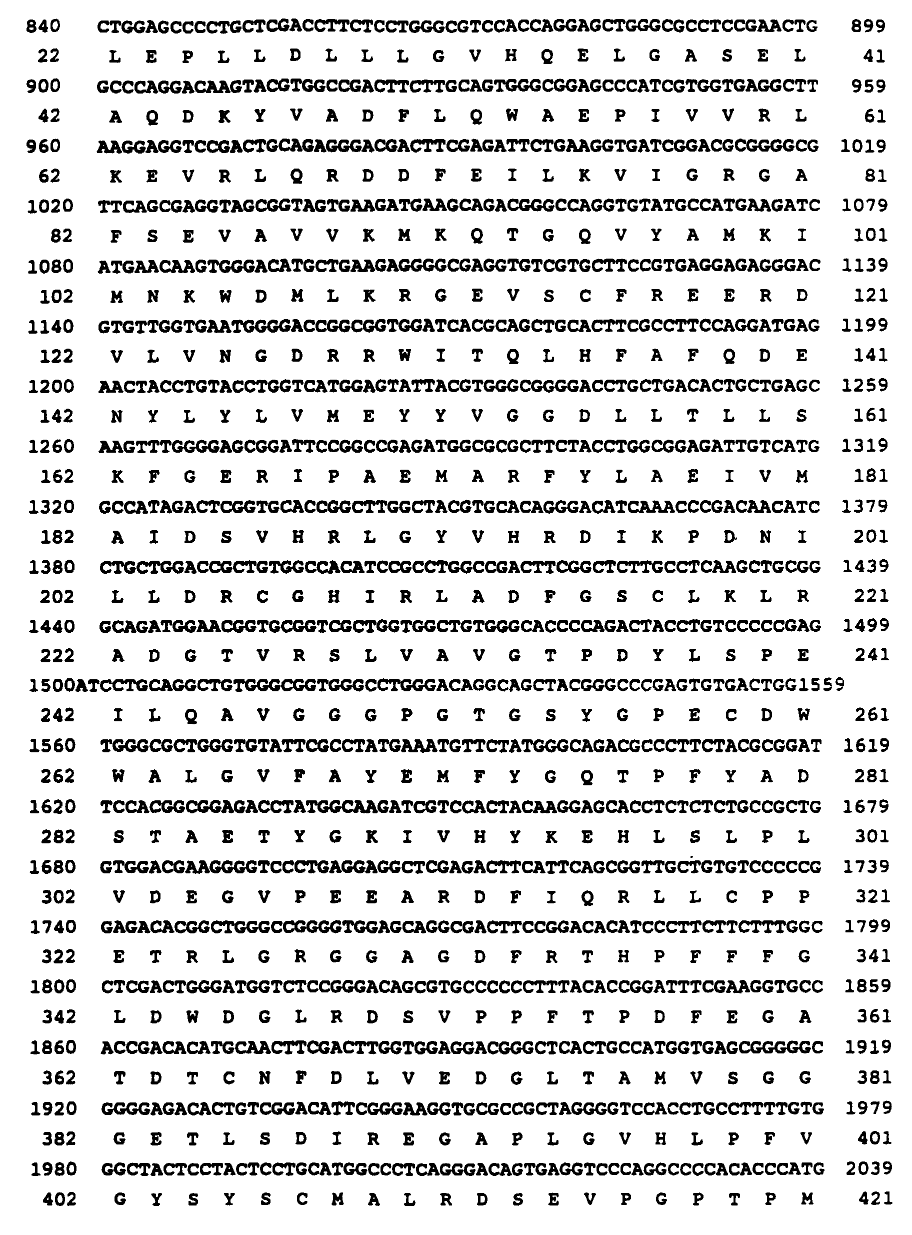

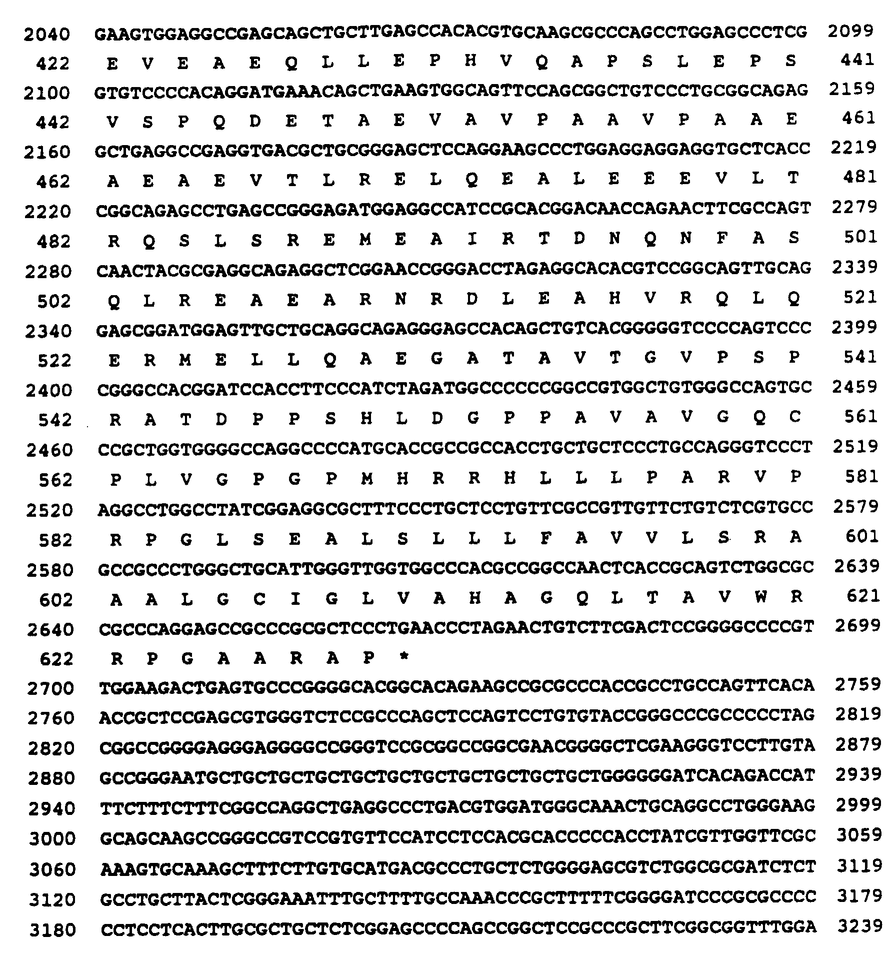

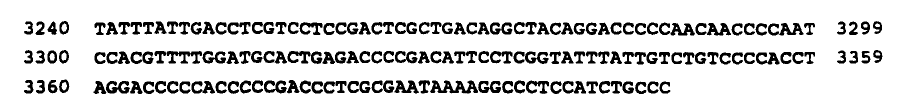

## Claims

1. A purified nucleic acid molecule comprising the nucleotide sequence of Figure 1b or the nucleotide sequence of Table I.

2. A purified nucleic acid molecule comprising a genomic fragment of a gene isolated from human chromosome 19q13, said nucleic acid molecule having the nucleotide sequence of Table I or the restriction map of Figure 1a, wherein site is 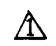 an insertion length polymorphism and site is 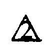 a variable length polymorphism associated with myotonic dystrophy.

3. A purified nucleic acid molecule according to claim 2, wherein said variable length polymorphism can expand said molecule by up to 10 kb by virtue of a CTG trinucleotide repeat.

4. A nucleotide probe selected from
(a) an oligonucleotide containing a trinucleotide repeat of at least 4 times, the trinucleotide being CTG;
(b) an oligonucleotide containing a trinucleotide repeat of 10 times, said trinucleotide being CTG;
(c) an oligonucleotide comprising a sequence of at least 12 nucleotides selected from the nucelotide sequence of claim 1; and
(d) an oligonucleotide comprising a sequence of at least 12 nucleotides selected from the nucleotide sequence of claim 2.

5. A method for screening a subject to determine if said subject is at risk for or affected with myotonic dystrophy, said method comprising:
(i) providing a biological sample containing DNA or RNA of said subject to be screened,
(ii) conducting a biological assay on said sample to detect the degree of expansion of a CTG trinucleotide repeat in a gene associated with myotonic dystrophy and derived from chromosome 19q13 of said biological sample, and
(iii) assessing the frequency of said trinucleotide repeat to determine at least susceptibility to myotonic dystrophy.

6. A method according to claim 5, wherein a determined frequency in step (iii) in excess of 40 repeats indicates a subject at risk for myotonic dystrophy and a determined frequency in excess of 50 repeats indicates a subject affected with myotonic dystrophy.

7. A method according to claim 5, wherein said biological assay of step (ii) includes amplification of a portion of said gene containing said trinucleotide repeat by a polymerase chain reaction technique using forward and reverse primers of appropriate primer sequence length selected from sequence portions of a nucleotide sequence of Figure 1a or of Figure 1b or of Table I.

8. A method according to claim 7, wherein, in step (ii), the degree of expansion is detected by use of a nucleotide probe which detects said CTG repeat and hybridizes to said amplified portion, determining the extent of said amplified portion to indicate the frequency of CTG repeats in said gene.

9. A method according to claim 5, wherein, in addition to detecting said CTG repeat, one or more polymorphisms of Table III are detected to complement said subject screening.

10. A kit for assaying for the presence of an expanded variable length polymorphism having a CTG trinucleotide repeat in a gene associated with myotonic dystrophy and derived from human chromosome 19q13, said kit comprising:
(i) a nucleotide probe which hybridizes to said variable length polymorphism, and is selected from those of claim 4, and
(ii) reagent means for detecting hybridization of said probe to said polymorphism,
said reagent means and said probe being provided in amounts sufficient to effect said assay.

## Patentansprüche

1. Gereinigtes Nucleinsäuremolekül, bestehend aus der Nucleotidsequenz der Figur 1b oder der Nucleotidsequenz der Tabelle 1.

2. Gereinigtes Nucleinsäuremolekül, bestehend aus einem Genomfragment eines aus dem menschlichen Chromosom 19q13 isolierten Gens, wobei das Nucleinsäuremolekül die Nucleotidsequenz der Tabelle 1 oder der Restriktionskarte in Figur 1a aufiveist , wobei die Stelle 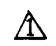 ein Insertionslängen-Polymorphismus und Stelle 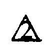 ein Polymorphismus variabler Länge ist, der mit myotonischer Dystrophie assoziiert wird.

3. Gereinigtes Nukleinsäuremolekül nach Anspruch 2, wobei der Polymorphismus variabler Länge das Molekül auf Grund der CTG-Trinucleotid-Replikation um bis zu 10 kb erweitern kann.

4. Nucleotidsonde, ausgewählt unter
(a) einem Oligonucleotid, das eine mindestens viermalige Trinucleotid-Replikation enthält, wobei das Trinucleotid CTG ist;
(b) einem Oligonucleotid, das eine mindestens zehnmalige Trinucleotid-Replikation enthält, wobei das Trinucleotid CTG ist;
(c) einem Oligonucleotid, das eine Sequenz von mindestens 12 Nucleotiden enthält, die aus der Nucleotidsequenz gemäß Anspruch 1 ausgewält sind;
(d) einem Oligonucleotid, das eine Sequenz von mindestens 12 Nucleotiden enthält, die aus der Nucleotidsequenz gemäß Anspruch 2 ausgewählt sind.

5. Methode zum Untersuchen einer Untersuchungsperson zur Bestimmung, ob diese Untersuchungsperson für myotonische Dystrophie anfällig oder davon befallen ist, wobei die Methode folgendes umfaßt:
(i) Bereitstellung einer biologischen Probe, die DNA oder RNA der zu untersuchenden Untersuchungsperson enthält,
(ii) Durchführung einer biologischen Untersuchung der Probe zur Bestimmung des Expansionsgrads einer CTG-Trinucleotid-Replikation in einem Gen, das mit myotonischer Dystrophie assoziiert wird und vom Chromosom 19q13 der biologischen Probe abgeleitet ist und
(iii) Beurteilung der Häufigkeit der Trinucleotid-Replikation zur Bestimmung zumindest der Anfälligkeit für myotonische Dystrophie

6. Methode nach Anspruch 5, bei der eine Bestimmung der Häufigkeit in Stufe (III) auf mehr als 40 Replikationen anzcigt, daß eine Untersuchungsperson für myotonische Dystrophie anfällig ist und eine Bestimmung der Häufigkeit auf über 50 Replikationen anzeigt, daß die Untersuchungsperson von myotonischer Dystrophie befallen ist.

7. Methode nach Anspruch 5, wobei die biologische Untersuchung der Stufe (ii) eine Amplifikation eines Teils des die Trinucleotid-Replikation enthaltenden Gens durch eine Polymerasekettenreaktionsmethode unter Zuhilfenahme von Vorwärts- und Rückwärtsprimern einer geeigneten Primersequenzlänge, ausgewählt aus Sequenzanteilen einer Nucleotidsequenz der Figur 1a oder Figur 1b oder Tabelle I, einschließt.

8. Methode nach Anspruch 7, bei der in Stufe (ii) der Expansionsgrad durch Verwendung einer Nuleotidsonde bestimmt wird, die die CTG-Replikation auffindet und in den amplifizierten Teil hybridisiert, wobei das Ausmaß des amplifizierten Teils bestimmt wird, um dadurch die Häufigkeit der CTG-Replikationen im Gen anzuzeigen.

9. Methode nach Anspruch 5, wobei zusätzlich zur Auffindung der CTG-Replikation, ein oder mehrere Polymorphismen der Tabelle III zur Ergänzung der Untersuchung der Untersuchungsperson bestimmt werden.

10. Kit zur Untersuchung auf Anwesenheit eines expandierten Polymorphismus variabler Länge mit einer CTG-Trinucleotid-Replikation in einem Gen, das als mit myotonischer Dystrophie assoziiert wird und aus dem menschlichen Chromosom 19q13 abgeleitet ist, wobei der Kit folgendes umfaßt:
i) eine Nucleotidsonde, die in den Polymorphismus variabler Länge hybridisiert und unter den in Anspruch 4 angegebenen ausgewählt wird, und
ii) Reagenzmittel zur Bestimmung der Hybridisierung der Sonde zum Polymorphismus,
wobei das Reagenzmittel und die Sonde in Mengen bereitgestellt werden, die zur Ausführung der Untersuchung ausreichen.

## Revendications

1. Une molécule d'acide nucléique purifié comportant la séquence de nucléotides de la figure 1b ou la séquence de nucléotides du tableau I.

2. Une molécule d'acide nucléique purifié comportant un fragment génomique d'un gêne isolé du chromosome humain 19q13, ladite molécule d'acide nucléique ayant la séquence de nucléotides du tableau I ou la carte de restriction de la figure 1a, dans laquelle le site 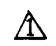 est un polymorphisme à longueur d'insertion et le site 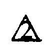 est un polymorphisme à longueur variable associé à la myotonie atrophique.

3. Une molécule d'acide nucléique purifié selon la revendication 2, dans laquelle ledit polymorphisme à longueur variable peut causer une expansion de ladite molécule jusqu'à 10 kb du fait d'une répétition d'un trinucléotide CTG.

4. Une sonde nucléotide sélectionnée parmi:
(a) un oligonucléotide contenant une répétition du trinucléotide 4 fois au moins, le trinucléotide étant CTG;
(b) un oligonucléotide contenant une répétition du trinucléotide 10 fois au moins, ledit trinucléotide étant CTG;
(c) un oligonucléotide comprenant une séquence de 12 nucléotides au moins, les nucléotides étant sélectionnés parmi la séquence de nucléotides de la revendication 1;
(d) un oligonucléotide comprenant une séquence de 12 nucléotides au moins, les nucléotides étant sélectionnés parmi la séquence de nucléotides de la revendication 2;

5. Une méthode de dépistage d'un sujet pour déterminer si ledit sujet est à risque ou atteint de myotonie atrophique, ladite méthode comportant:
(i) l'élaboration d'un échantillon biologique contenant l'ADN ou l'ARN du dit sujet à dépister,
(ii) la réalisation d'une analyse biologique du dit échantillon pour détecter le degré d'expansion d'une répétition de trinucléotides CTG au sein d'un gène associé à la myotonie atrophique et dérivé du chromosome 19q13 du dit échantillon biologique, et
(iii) l'évaluation de la fréquence de ladite répétition des trinucléotides pour déterminer au moins la susceptibilité à la myotonie atrophique.

6. Une méthode selon la revendication 5, selon laquelle une fréquence, déterminée à l'étape (iii), excédant 40 répétitions indique un sujet à risque de myotonie atrophique et une fréquence déterminée excédant 50 répétitions indique un sujet atteint de myotonie atrophique.

7. Une méthode selon la revendication 5, selon laquelle ladite analyse biologique de l'étape (ii) inclut l'amplification d'une portion du dit gène contenant ladite répétition de trinucléotides au moyen d'une technique de réaction en chaîne de polymérases utilisant des amorces directes et inverses à séquence d'amorce de longueur appropriée sélectionnées parmi des portions de séquence d'une séquence de nucléotides de la figure 1a, ou de la figure 1b, ou du tableau I.

8. Une méthode selon la revendication 7, selon laquelle, à l'étape (ii), le degré d'expansion est détecté au moyen d'une sonde nucléotide qui détecte ladite répétition de CTG et s'hybridise avec ladite portion amplifiée, déterminant l'importance de ladite portion amplifiée pour indiquer la fréquence des répétitions de CTG dans ledit gène.

9. Une méthode selon la revendication 5, selon laquelle, outre la détection de ladite répétition de CTG, un ou plusieurs polymorphismes du tableau III sont détectés pour complémenter le dépistage du dit sujet.

10. Un coffret d'essai pour déterminer la présence d'un polymorphisme à longueur variable en expansion ayant une répétition de trinucléotides CTG dans un gène associé à la myotonie atrophique et dérivé du chromosome humain 10q13, ledit coffret contenant:
(i) une sonde à nucléotides qui s'hybridise avec ledit polymorphisme de longueur variable, et est sélectionnée parmi celles de la revendication 4, et
(ii) un réactif pour la détection de l'hybridisation de ladite sonde avec ledit polymorphisme,
ledit réactif et ladite sonde étant fournis en quantités suffisantes pour mener ladite analyse.
